# EUROPEAN PATENT SPECIFICATION

(11) **EP 2 488 872 B1**
(45) Date of publication and mention of the grant of the patent: **16.04.2014**
(21) Application number: 10768694.1
(22) Date of filing: 13.10.2010
(51) Int. Cl.: G01N 33/569

(54) **METHODS FOR PRRSV DETECTION**
VERFAHREN ZUR PRRSV-DETEKTION
PROCÉDÉS POUR DÉTECTION DE PRRSV

(30) Priority: 13.10.2009 EP 09012930
(43) Date of publication of application: 22.08.2012
(73) Proprietor: Fraunhofer-Gesellschaft zur Förderung der angewandten Forschung e.V., 80686 München (DE)
(72) Inventor: ULBERT, Sebastian, 04277 Leipzig (DE); GIESE, Matthias, 69123 Heidelberg (DE); AL-ROBAIY, Samiya, 04289 Leipzig (DE); DELAROQUE, N, 04317 Leipzig (DE); SALOMO, Mathias, 04277 Leipzig (DE)
(86) International application number: PCT/EP2010/006274
(87) International publication number: WO 2011/045056

(56) References cited:
- WO-A2-2007/006031
- GB-A- 2 289 279
- US-A1- 2006 234 211
- US-A1- 2007 003 570
- BROWN ELIZABETH ET AL: "Antibody Response to Porcine Reproductive and Respiratory Syndrome Virus (PRRSV) Nonstructural Proteins and Implications for Diagnostic Detection and Differentiation of PRRSV Types I and II", CLINICAL AND VACCINE IMMUNOLOGY, vol. 16, no. 5, May 2009 (2009-05), pages 628-635, XP002609345, ISSN: 1556-6811
- SEUBERLICH T ET AL: "Nucleocapsid Protein-Based Enzyme-Linked Immunosorbent Assay for Detection and Differentiation of Antibodies Against European and North American Porcine Reproductive and Respiratory Syndrome Virus", CLINICAL AND DIAGNOSTIC LABORATORY IMMUNOLOGY, AMERICAN SOCIETY FOR MICROBIOLOGY, US, vol. 9, no. 6, 1 November 2002 (2002-11-01), pages 1183-1191, XP003010089, ISSN: 1071-412X
- DENAC ET AL: "An indirect ELISA for the detection of antibodies against porcine reproductive and respiratory syndrome virus using recombinant nucleocapsid protein as antigen", JOURNAL OF VIROLOGICAL METHODS, ELSEVIER BV, NL, vol. 65, no. 2, 1 May 1997 (1997-05-01), pages 169-181, XP005538368, ISSN: 0166-0934
- DEA S ET AL: "Competitive ELISA for detection of antibodies to porcine reproductive and respiratory syndrome virus using recombinant E. coli-expressed nucleocapsid protein as antigen", JOURNAL OF VIROLOGICAL METHODS 200006 NL, vol. 87, no. 1-2, June 2000 (2000-06), pages 109-122, XP002570839, ISSN: 0166-0934
- WITTE S B ET AL: "Development of a recombinant nucleoprotein-based enzyme-linked I'mmunosorbent assay for quantification of antibodies against porcine reproductive and respiratory syndrome virus", CLINICAL AND DIAGNOSTIC LABORATORY IMMUNOLOGY 200007 US, vol. 7, no. 4, July 2000 (2000-07), pages 700-702, XP002570840, ISSN: 1071-412X

## Description

### FIELD OF THE INVENTION

The present invention relates to the field of PRRSV detection. In particular, the present invention discloses methods for the detection of PRRSV antibodies in serum. The present invention furthermore discloses systems and kits that may be used in the disclosed methods.

### BACKGROUND OF THE INVENTION

Arteriviruses are well-known to play a key role in various diseases associated with livestock. The family *Arteriviridae* generally comprises four enveloped, positive-stranded RNA viruses having an icosahedral core: equine arteritis virus, lactate dehydrogenate elevating virus of mice, porcine reproductive and respiratory syndrome virus, and simian hemorrhagic fever virus.

Porcine reproductive and respiratory syndrome virus (PRRSV), also known as Blue-Ear Pig Disease, is of particular importance since it causes a disease in pigs, called porcine reproductive and respiratory syndrome (PRRS). This economically devastating pandemic disease causes reproductive failure in breeding stock and respiratory tract illness in young pigs. Initially referred to as "mystery swine disease" and "mystery reproductive syndrome," this disease was first reported in North America and Central Europe in 1987. PRRS is the economically most important pig-related disease, with estimated losses in the United States swine industry amounting to $460 million annually.

Given the commercial implications of PRRS, a means for the fast and reliable detection of PRRSV infections is of paramount importance to, for example, preventing and containing the spread of this infection as quickly as possible. However, while there are a few known detection systems suitable for screening for the presence of a PRRS infection, these detection systems generally suffer from insufficient sensitivity and/or specificity allowing for a quick and reliable viral detection, thus leading to an unacceptably high occurrence of false-negative or false-positive screening results.

US 2007/0003570 provides methods and materials to determine whether or not an organism contains anti-PRRSV antibodies.

Brown et al (Clin. Vaccine Immunol., 2009, 16, 628-635) use a dual NSp7 ELISA to differentiate PRRSV type I and II.

Thus, there exists a need for improved methods and kits for the detection of PRRSV infections and systems and kits useful for carrying out these methods.

### SUMMARY OF THE INVENTION

It is an object of the invention to provide methods for the detection of porcine reproductive and respiratory syndrome virus (PRRSV) antibodies, including the steps of providing at least two PRRSV proteins wherein one of the at least two PRRSV proteins is the GP5 protein, and wherein one of the at least two PRRSV proteins is the NSp7 protein or fragments thereof that are recognized as an epitope by a PRRSV antibody and have a length of at least 10 continuous amino acids; contacting the at least two PRRSV proteins or fragments thereof with a liquid sample that is derivable from an individual to be tested under conditions to allow the binding of PRRSV anti-bodies comprised by the samples to the PRRSV proteins; and detecting the PRRSV antibodies bound to the PRRSV proteins.

Advantageously, the present invention is based on the unexpected finding that a combination of more than one PRRSV protein provides an improved and reliable detection of PRRSV antibodies in a liquid sample that is derivable from an individual to be tested. Thus, an improved monitoring of the presence and progress of a PRRSV infection or an effective PRRSV vaccination is possible. Unexpectedly, it also discovered that using more than a single PRRSV protein provides a more sensitive and specific method for detecting PRRSV. A higher sensitivity naturally results in a larger number of correctly determined PRRSV positives, while a higher specificity naturally results in a lower number of PRRSV false-positives. In one embodiment, a combination of three PRRSV proteins is utilized, comprising both GP5 and NSp7, and an additional protein selected from the group consisting of the M-protein, the E-protein, GP3 and the N-protein.

The inventors have found that unexpectedly, a particularly high sensitivity can be achieved when a combination of the three PRRSV proteins NSp7, GP5 and GP3 protein is used for the detection of PRRSV. An ELISA assay according to one embodiment of the present invention comprising these three proteins shows a superior sensitivity when compared with other, commercially available ELISA test systems for PRRSV, and is especially well suited also for the detection of PRRSV in highly diluted samples.

The inventors have found that the antigens that are capable of eliciting the highest antibody signal in a PRRSV ELISA assay are GP5, GP3 and NSP7. Unexpectedly, a clear synergistic effect is seen with a combination of the individual antigens is analysed in a PRRSV ELISA according to one embodiment of the present invention, comprising antigens of the SEQ IDs 1 and 9 (GP5), SEQ IDs 5 and 13 (GP3) and SEQ IDs 19 and 25 (NSP7).

In a large series of PRRSV-positive pig sera, the inventors could demonstrate that the antigens that elicit the strongest signal in a PRRSV ELISA vary significantly between different samples. It has been found that in some, NSP 7 is the dominant antigen, whereas in other samples, GP3 or GP5 are the antigens that are predominantly recognized. Therefore, to detect all the PRRSV-positive samples correctly as positive, the presence of all three of these antigens is highly beneficial. Thus, the highest sensitivity was observed when an ELISA comprising NSP7 (SEQ ID NO:19 and 25), GP3 (SEQ ID NO:5 and 13) and GP5 (SEQ IDNO:1 and 9) was utilized. Hence, due to the high degree of variability in the anti-PRRSV antibody responses of pigs, a clear synergistic effect is seen by using the mixture of antigens according to one embodiment of the present invention, namely NSP7, GP5 and GP3 of both genotypes. The presence of all three of these antigens is essential to detect the maximum numbers of PRRSV infections. This leads to a significant advantage over available technologies relying only on one antigen or on antigen mixtures different from the one used in the present invention. Furthermore, the ELISA comprising SEQ IDs 1 and 9 (GP5), SEQ IDs 5 and 13 (GP3) and SEQ IDs 19 and 25 (NSP7) proved to be more sensitive than ELISA assays comprising different combinations of structural and non-structural PRRSV proteins.

In another embodiment, the at least one of the PRRSV proteins is derived from the European PRRSV strain Lelystad or the US PRRSV strain VR-233 and/or further comprises an MBP-tag and/or a His-tag.

In yet another embodiment, the detection step is carried out immunologically and/or the detection of PRRSV antibodies indicate a PRRSV infection or an effective PRRSV vaccination.

The present invention further contemplates a system for the detection of PRRSV antibodies, comprising a support, wherein immobilized on the support are at least two PRRSV proteins wherein one of the at least two PRRSV proteins is the GP5 protein, and wherein one of the at least two PRRSV proteins is the NSp7 protein, or fragments thereof that are recognized as an epitope by a PRRSV antibody and have a length of at least 10 continuous amino acids.

In another aspect of the invention, a kit comprising at least two PRRSV proteins wherein one of the at least two PRRSV proteins is the GP5 protein, and wherein one of the at least two PRRSV proteins is the NSp7 protein, or fragments thereof that are recognized as an epitope by a PRRSV antibody and have a length of at least 10 continuous amino acids is provided.

In both the systems and kits described herein, the provided proteins may be selected from the group consisting of a fragment of the GP5 protein, preferably SEQ ID NO: 1 or 9, the NSp7 protein, preferably SEQ ID NO: 19 or 25, and a fragment of the GP3 protein, preferably SEQ ID NO: 5 or 13.

### BRIEF DESCRIPTION OF THE DRAWINGS

The present invention is in its broadest sense defined by the appended claims. The invention, however, both as to organization and method of operation, together with objects, features, and advantages thereof, may best be understood by reference to the following detailed description when read with the accompanying drawings in which:
FIG. 1 shows the plasmid map of the pMAL Tag2 vector (SEQ ID NO: 27)
FIG. 2 depicts the results of an ELISA assay utilizing an expressed GP5 protein.
FIG. 3 depicts the results of an ELISA assay using multiple PRRSV proteins and a comparison with an IDEXX ELISA.
FIG. 4 depicts the result of an ELISA assay using multiple PRRSV proteins (FIG. 4 A) and of IDEXX ELISA (FIG. 4 B)
FIG. 5 depicts the results of an ELISA assay using multiple PRRSV proteins

### DETAILED DESCRIPTION OF EMBODIMENTS

The present invention is based on the unexpected finding that a combination of more than one PRRSV protein, or fragments thereof, provides for an efficient and reliable detection of PRRSV antibodies in a liquid sample that is derivable from an individual to be tested. Unexpectedly, it was discovered that, due to the inclusion of more than a single protein according to the present methods, higher antibody signals, and thus more reliable results, can be obtained compared to those methods where only a single protein is present. Advantageously, the method according to the invention furthermore allows for a safer distinction between the various possible PRRSV strains infecting an animal, as well as for providing valuable information about the state of a detected PRRSV infection.

PRRSV is a small, enveloped RNA virus. It contains a single-stranded, positive-sense, RNA genome with a size of approximately 15 kilobases. The genome codes for nine open reading frames. PRRSV is a member of the genus *Arterivirus*, family *Arteriviridae,* and the order nidovirales. An example for a North American strain is VR-2332 and the European strain is known as the Lelystad Virus (LV). The European and North American PRRSV strains have been described to manifest similar clinical symptoms, but they represent two distinct viral genotypes whose genomes diverge by approximately 40%. The genetic variation among the viruses isolated from different locations further increases the difficulty of reliably detecting an infection caused by the respective strains.

The term "PRRSV antibody" as used herein, refers to any antibody that recognizes PRRSV, that is, at least one component of PRRSV, as an epitope. In other words, the term "PRRSV antibody" refers to an anti-PRRSV antibody, for example, produced by a pig in response to an infection with PRRSV. Preferably, the PRRSV antibody recognizes GP3, the M-protein, the E-protein or the N-protein in addition to NSp7 protein and GP5 protein of PRRSV and/or fragments thereof as an epitope. Especially pre-ferred fragments of the European strain proteins are aa 33-201 (SEQ ID NO: 1) or aa 33-66 of the GP5 protein (SEQ ID NO: 3), aa 55-238 of the GP3 protein (SEQ ID NO: 5), aa 100-173 of the M protein (SEQ ID NO: 7). Especially preferred fragments of the North American strain are aa 66-200 (SEQ ID NO: 9) or aa 26-67 (SEQ ID NO: 11) of the GP5 protein, aa 61-201 of the GP3 protein (SEQ ID NO: 13), or aa 97-174 of the M protein (SEQ ID NO: 15).

Whenever reference is made to a PRRSV protein, the full-length protein and the fragment of said protein are both referred to, unless explicitly stated otherwise. For example, the term "GP5 protein" designates both the full-length PRRSV GP5 protein and a fragment of said GP5 protein that is sufficiently recognized as an epitope by a PRRSV antibody. Furthermore, unless designated otherwise, proteins of both strains, the European and the North American are referred to.

The sequences of the full-length proteins ORF7, NSp7 and E protein of the European strain are set forth in SEQ ID NO: 17, 19 and 21, respectively. The sequences of the full-length proteins ORF7 and NSp7 of the North American strain are set forth in SEQ ID NO: 23 and 25, respectively.

A "fragment of a PRRSV protein" as used herein, refers to any portion of a PRRSV protein that is recognized by the immune system of the PRRSV host, i.e. which is recognized as an epitope by a PRRSV antibody, preferably by the porcine immune system, and has a length of at least 10 continuous amino acids. In other embodiments, the fragment of the PRRSV protein has a length of ≥ 15 continuous amino acids, ≥ 20 continuous amino acids, ≥ 25 continuous amino acids, ≥ 30 continuous amino acids or ≥ 35 continuous amino acids. In more preferred embodiments, the fragment of the PRRSV protein has a length of ≥ 30 and ≤ 40, 34, or 35 continuous amino acids.

In one aspect, the present invention is directed to a method for the detection of porcine reproductive and respiratory syndrome virus (PRRSV) antibodies comprising the steps of: a) providing at least two PRRSV proteins wherein one of the at least two PRRSV proteins is the GP5 protein, and wherein one of the at least two PRRSV proteins is the NSp7 protein or fragments thereof that are recognized as an epitope by a PRRSV antibody and have a length of at least 10 continuous amino acids; b) contacting the at least two PRRSV proteins or fragments thereof with a liquid sample that is derivable from an individual to be tested under conditions sufficient to allow the binding of PRRSV antibodies in the liquid sample that is derivable from an individual to be tested to the PRRSV proteins or fragments thereof; and c) detecting the PRRSV antibodies that are bound to the PRRSV proteins or fragments thereof.

In a first step according to the inventive methods, at least two PRRSV proteins are provided in order to detect PRRSV antibodies. Preferably, the PRRSV proteins are provided in an immobilized form, which has an advantage that the detection of PRRSV antibodies bound to said proteins can be accomplished more readily. In one preferred embodiment, the at least two PRRSV proteins are immobilized on a solid support, wherein any solid support suitable for this purpose and known to the skilled person can be utilized, such as a membrane, or a gel matrix. Most preferably, the solid support is a plate, for example, a 96-well microtiter plate, as commonly used in cell culture or with enzyme-linked immunosorbent assays (ELISA) techniques. This embodiment has the advantage that the plate can be readily coated with the PRRSV proteins by the skilled person, and that the binding of PRRSV antibodies to PRRSV proteins can be easily detected using standard laboratory equipment, such as an ELISA plate reader.

In one embodiment of the invention, the wells contained within the well plate are coated with the at least two PRRSV proteins at different concentrations, wherein preferably a serial dilution is carried out. In another embodiment, the wells are coated with PRRSV proteins from the different PRRSV strains, for example, one well contains at least two PRRSV proteins from only the European Lelystad strain. This arrangement advantageously allows a swift differentiation between the various PRRSV strains.

In a next step according to the invention, the at least two PRRSV proteins are brought into contact with a liquid sample that is derivable from an individual to be tested under conditions that allow the binding of PRRSV antibodies, which are present in the liquid sample that is derivable from an individual to be tested, upon infection of the host with PRRSV. Conditions affecting the binding of antibodies to proteins are known to the skilled person, as are standard protocols for facilitating the binding between the two components. Such conditions can include the temperature, duration of contacting, presence and concentration of blocking agents and/or detergents and so on. In one embodiment, standard ELISA techniques are employed.

In the following, a specific, yet generally applicable embodiment of the invention is described:
In a first step, the PRRSV proteins and a negative control, preferably having the same amount of Maltose Binding Protein (MBP), are diluted to appropriate concentrations in a buffer, preferably the buffer is a 50 mM Na₂CO₃ buffer. 100 µl volumes of such diluted antigen are then dispensed into the wells of an ELISA microplate, preferably a flat-bottomed, high protein binding ELISA microplate (Polysorp, Nunc 475094). Even more preferably, 2 wells per test sample are used as well as 2 wells for the negative control reaction. The microplate is then sealed and incubated overnight at 4°C. Thereafter the plates are washed, preferably 3 times with 1x PBS-Tween 0.05%.

In an additional step step, 200 µl blocking buffer, preferably PBS with 5% low-fat milk, is added. The microplates are then sealed and incubated, preferably under movement, for example on an on an orbital shaker. This incubation is preferably carried out for 2 h at room temperature.

In a further step, the plates are washed, preferably for 3 times with 1x PBS-Tween 0.05%.

In turn, the samples to be tested are diluted, for example 50 times, in the blocking buffer and dispensed in each test well, wherein a volume of 100 µl is preferred. The microplates are again incubated, likewise preferably under movement, for example, on an orbital shaker for 1 hour at room temperature.

Following the incubation step, the plates are washed, preferably 3 times with 1x PBS-Tween 0.05%.

In a next step, the working dilution of a secondary antibody, preferably a rabbit anti-pig immunoglobulin horseradish peroxidise conjugate (1:10,000 Sigma A5670), is prepared in blocking buffer and 100 µl are added to each test well. In turn, the microplates are incubated likewise, preferably under movement, for example, on an orbital shaker for 1 hour at room temperature.

After incubation, the plates are washed as described above and immediately re-filled with substrate solution, preferably 100 µl TMB One Solution (Promega G7431) followed by incubation, preferably at room temperature for 30 min in a dark environment without shaking. 50 µl of stopping solution, preferably consisting of 1 M sulphuric acid is then added.

Finally, the resulting absorbance values are measured, preferably utilizing an ELISA reader at 450 nm and 520 nm as a reference.

The sample may be any serum sample suitable for the detection of PRRSV antibodies and generally encompasses any liquid that is derivable from an individual to be tested. Preferably, the serum sample is selected from the group consisting of blood, serum, sputum, saliva and/or milk. More preferably, the serum sample is porcine serum. The serum sample has been isolated from the animal prior to carrying out the method of the present invention. While the isolated serum may be subjected to further purification and/or treatment steps in order to improve the binding and/or detection of the PRRSV antibodies, it has been found that such further steps are not necessary in order to achieve reliable results. Therefore, the present invention advantageously offers the possibility of carrying out the present detection method 'on the spot', i.e. directly at the site of a suspected PRRSV infection.

In a further step according to present invention, the PRRSV antibodies bound to the PRRSV proteins are detected. Detection of said PRRSV antibodies can be carried out by any means known to the skilled person. Preferably, the PRRSV antibodies are detected by immunological means, e.g. via conjugated secondary antibodies or functional fragments thereof, such as F_{ab} fragments. Such conjugated secondary antibodies are directed against the antibody molecules of the host, for example, against porcine antibodies. Generally such antibodies are generated in a different species, e.g. in rabbit, and recognize the species-specific portion(s) of the target antibodies as an epitope. Such secondary antibodies may be conjugated to an enzyme, such as horseradish peroxidase, to enable conversion of a substrate resulting in chemoluminescence or leading to a change in color. Likewise they may be labeled, e.g. by an isotope and/or any other detectable label known to the skilled person. Advantageously, both PRRSV proteins present in the method according to the invention will allow the binding of PRRSV antibodies. In turn, the signal generated by the secondary antibodies binding to the primary antibodies will be amplified, thus increasing the sensitivity of the method according to the invention.

In a further embodiment of the invention, the at least two PRRSV proteins are selected from the group consisting of GP3, the M-protein, the E-protein or the N-protein in addition to GP5 and NSp7.

In another embodiment of the invention, at least one of the PRRSV proteins employed is a recombinant protein. Preferably, more than one, two, three, four, five and/or all of the PRRSV proteins are recombinant proteins. Recombinant expression of proteins is a technique commonly known to the skilled person. Various expression systems and/or hosts are known, e.g. recombinant expression in yeast, mammalian cells or bacteria. Thus, the provision of the at least two PRRSV proteins according to the method of the invention may furthermore comprise a step of recombinantly expressing and/or purifying the PRRSV protein(s). In one embodiment, the PRRSV protein(s) is/are efficiently expressed in bacteria as N-terminal fusion proteins with the Maltose Binding Protein (MBP) from *E. coli.* Preferably, the PRRSV protein(s) additionally comprise a C-terminal His-tag. This tag confers an advantage that the expressed proteins can be readily purified, and to extremely high grades, preferably using both tags in a tandem-protocol. It is possible to rapidly remove the MBP-tag by digestion with recombinant Factor X. However, unexpectedly it has been found that MBP is completely inert and is not recognized by porcine serum, hence the presence of MBP in the reaction vessel does not lead to false positive results. The skilled person will be aware of the fact that further different tagging systems are effective for achieving a purification of the PRRSV proteins.

In another embodiment of the invention, at least one of the PRRSV proteins is derived from the European PRRSV strain Lelystad (Genbank accession number: M96262) or a North American PRRSV strain e.g. VR-2332 (Genbank accession number: EF536003). Preferably, all of the at least two PRRSV proteins are derived from the same PRRSV strain.

The GP5 protein or sequence can be the GP5 sequence or protein of Acc No. AF095507 (strain 18027), ADN42860 (strain GDENP/1/08) or ACU56734 (strain 08-1665-YAS).

The NSp7 sequence or protein can be the NSp7 sequence or protein from strain VR2332, ABY66100 (strain rV63) or ACP43262 (strain clone 20).

The GP3 sequence or protein can be the GP3 sequence or protein from VR2332 or ACY29601

In the present invention, one of the at least two PRRSV proteins is the GP5 protein. Unexpectedly, it has been found that GP5 may result in a higher antibody signal compared to the N protein. Thus, if GP5 is used as one of the at least two PRRSV proteins, a higher sensitivity of the assay advantageously results.

Furthermore, in the present invention, one of the at least two PRRSV proteins is NSp7. Similar to GP5, it has been found that the use of NSp7 may result in a higher antibody signal compared to that associated with the N protein. Thus, if NSp7 is used as one of the at least two PRRSV proteins, a higher sensitivity of the assay advantageously results.

Thus, in the present invention, the at least two PRRSV proteins comprise GP5 as well as NSp7. As explained above, both proteins result in a higher sensitivity of the assay. It was found that if both proteins are combined together in a detection assay, a beneficial synergetic effect will occur.

More preferably, a combination of three or four proteins comprising GP5 and NSp7 as well as at least one protein selected from the group consisting of M-protein, the E-protein, GP3 and the N-protein is utilized. Such a combination of three PRRSV proteins is more preferred than a combination of four proteins. Even more preferably, a combination of the three PRRSV proteins GP5, NSp7 and GP3 is used. The GP5 protein is preferably selected from the group consisting of a fragment of the GP5 protein of the European or North American strain, aa 33-201 of the GP5 protein of the European strain (SEQ ID NO: 1) and aa 26-200 of the GP5 protein of the North American strain (SEQ ID NO: 9). The GP3 protein is preferably selected from the group consisting of a fragment of the GP3 protein of the European or North American strain, aa 55-238 of the GP3 protein of the European strain (SEQ ID NO: 5) and aa 61-201 of the GP3 protein of the North American strain (SEQ ID NO: 13). Additional combinations of four, five or six of said PRRSV proteins are likewise encompassed.

In a further embodiment of the present invention, the detection of PRRSV antibodies indicates the presence of a PRRSV infection or an effective PRRSV vaccination. Thus, if PRRSV antibodies are detected in the liquid sample that is derivable from an individual to be tested, a PRRSV infection may be actually present or may have been present in the past. In a preferred embodiment of the present methods, pieces of information regarding the state of the PRRSV infection are derivable. This has the advantage that it becomes possible to differentiate whether a PRRSV infection is in an onset phase, has manifested itself, or is already subsiding. This differentiation may be carried out taking into account the various Ig-subclasses. In addition, it has unexpectedly been found that the antibodies directed against GP5, preferably directed against the C-terminus of GP5, and more preferably those directed against the fragment as set forth in SEQ ID NO: 1 and/or 9, are among the first to be produced by an infected individual, and furthermore are among the first to stop being produced by the individual. In comparison, antibodies directed against the N protein likewise are produced quickly, but tend to be produced for an extended period of time during the infection. Thus, detection of antibodies against GP5 indicates a recent occurrence of a PRRSV infection.

In a further aspect, the present invention is directed to a system for the detection of PRRSV antibodies, wherein the system comprises a support, and immobilized thereon at least two PRRSV proteins, wherein one of the at least two PRRSV proteins is the GP5 protein, and wherein one of the at least two PRRSV proteins is the NSp7 protein, or fragments thereof that are recognized as an epitope by a PRRSV antibody and have a length of at least 10 continuous amino acids.

It will be readily apparent to the skilled person that the features described above in connection with the methods of the present invention can be easily adapted into features of the system according to the invention. This observation is particularly true for the PRRSV proteins immobilized on the support.

In the present invention, the at least two PRRSV proteins immobilized on the support comprise GP5 and NSp7. As explained above, both proteins yield an increased sensitivity of the assay. It was found that if both proteins are combined in an assay, an advantageous synergetic effect will occur.

In one preferred embodiment according to the invention, a combination of three proteins comprising GP5 and NSp7, as well as at least one protein selected from the group consisting of M-protein, the E-protein, GP3 and the N-protein, is utilized. More preferably, a combination of the three PRRSV proteins GP5, NSp7 and GP3 is used. The GP5 protein is preferably selected from the group consisting of a fragment of the GP5 protein of the European or North American strain, aa 33-201 of the GP5 protein of the European strain (SEQ ID NO: 1) and aa 26-200 of the GP5 protein of the North American strain (SEQ ID NO: 9). The GP3 protein is preferably selected from the group consisting of a fragment of the GP3 protein of the European or North American strain, aa 55-238 of the GP3 protein of the European strain (SEQ ID NO: 5) and aa 61-201 of the GP3 protein of the North American strain (SEQ ID NO: 13). Additional combinations of four, five or six of said PRRSV proteins are likewise encompassed. .

The support can be any suitable support known to the skilled person. Preferably, the support is suitable for the use in immunological techniques, such as an ELISA technique. In one embodiment of the invention, the support is a solid support, preferably a microtiter plate.

The system may additionally comprise a microtiter plate reader. Such readers are commonly know to the skilled person and readily available.

In another aspect, the present invention is directed to a kit for the detection of PRRSV antibodies, wherein the kit comprises at least two PRRSV proteins wherein one of the at least two PRRSV proteins in the GP5 protein, and wherein one of the at least two PRRSV proteins is the NSp7 protein, or fragments thereof that are recognized as an epitope by a PRRSV antibody and have a length of at least 10 continuous amino acids.

As explained above, it will be readily apparent to the skilled person that the features described above in connection with the method or system of the present invention can be easily adapted into features of the kit according to the invention. This observation is particularly true for the PRRSV proteins contained in the kit. Therefore, some of the preferred embodiments applicable for the kit are as follows:

In one embodiment, at least three PRRSV proteins are comprised by the kit, i.e. a combination of three proteins comprising GP5 and NSp7 and at least one protein selected from the group consisting of GP3, the M-protein, the E-protein, and the N-protein is preferred. More preferably, a combination of the three PRRSV proteins GP5, NSp7 and GP3 is contained in the kit. The GP5 protein is preferably selected from the group consisting of a fragment of the GP5 protein of the European or North American strain, aa 33-201 of the GP5 protein of the European strain (SEQ ID NO: 1) and aa 26-200 of the GP5 protein of the North American strain (SEQ ID NO: 9). The GP3 protein is preferably selected from the group consisting of a fragment of the GP3 protein of the European or North American strain, aa 55-238 of the GP3 protein of the European strain (SEQ ID NO: 5) and aa 61-201 of the GP3 protein of the North American strain (SEQ ID NO: 13). Additional combinations of four, five or six of said PRRSV proteins are likewise encompassed.

The kit may additionally comprise a support. The support can be any suitable support known to the skilled person. Preferably, the support is suitable for the use in immunological techniques, such as the ELISA technique, e.g. the support is a solid support, preferably a microtiter plate.

The kit may additionally comprise reagents used for immunological techniques. Preferably, such reagents include buffers, secondary antibodies, preferably labeled and/or conjugated and substrates for the detection reaction, such as TMB or DAB.

Further advantageous embodiments of the present invention are provided in the dependent claims.

### EXAMPLES

### EXAMPLE 1: Expression of recombinant PRRSV proteins

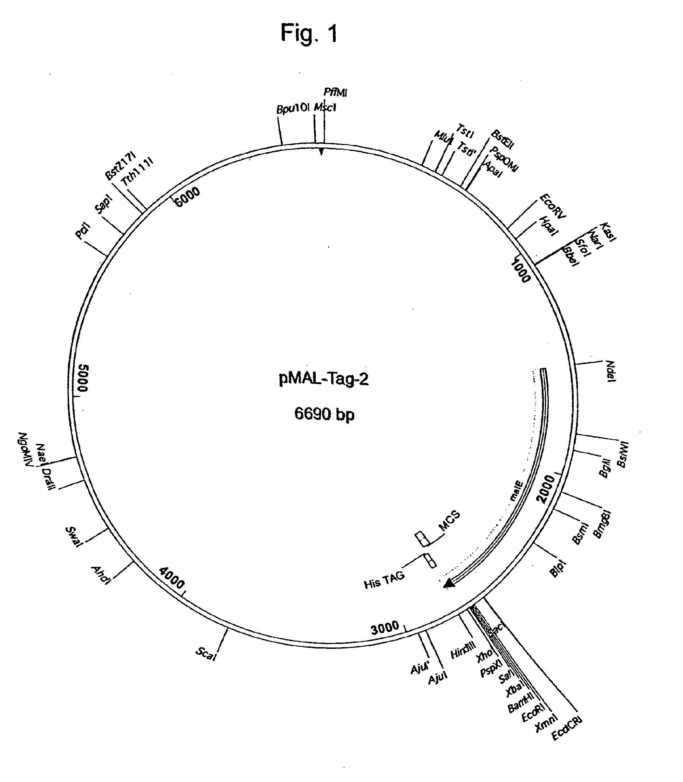

Chemically competent E. *coli* cells (Rosetta DE3) were transformed with the vector pMal Tag2 containing the PRRSV gene of interest. The vector pMal Tag2 (SEQ ID NO: 27) is a modification of the pMal™-c2X plasmid (New England Biolabs) such that a C-terminal His-tag is added to the MBP-fusion protein. FIG. 1 shows a plasmid map of the vector.

The sequence of MBP is known (*E.coli;* GenBank accession AAB86559). For reasons of convenience, it is presently set forth herein at SEQ ID NO: 28.

TABLE 1 summarizes those DNA sequences that were cloned into the pMal Tag2 vector at the indicated positions for expressing the various PRRSV proteins. The first column designates the name of the expressed PRRSV protein: Proteins/cDNA derived from the European PRRSV strain are designated with an "EU", proteins/cDNA derived from the North American PRRSV strain are designated with a "VR". The second column designates the position and restriction sites of the pMal Tag2 vector used to insert the PRRSV cDNA. The third column designates whether the PRRSV protein was expressed as a full-length protein or as a fraction thereof. The remaining columns designate the SEQ ID NOs of the expressed protein sequence and the cDNA sequence cloned into the vector, respectively.

**TABLE 1**

| **Protein** | **Restriction site** | **Expressed Region** | **Prot. Seq.** | **cDNA Seq.** |
|---|---|---|---|---|
| EU-ORF7 | BamH1 (2701) /Xho1 (2725) | Full-length | SEQ ID NO:17 | SEQ ID NO:18 |
| EU-NSP7 | EcoR1 (2695) /Sal1 (2713 | Full-length | SEQ ID NO:19 | SEQ ID NO:20 |
| EU-GP5-CE | BamH1 (2701) /Xho1 (2725) | aa 33-201 | SEQ ID NO:1 | SEQ ID NO:2 |
| EU-GP5-ecto | BamH1 (2701) /Xho1 (2725) | aa 33-66 | SEQ ID NO:3 | SEQ ID NO:4 |
| EU GP3-Mid-2 | BamHI(2701)/ Sal1 (2713) | aa 55-238 | SEQ ID NO:5 | SEQ ID NO:6 |
| EU-E-Protein | BamH1 (2701) /Xho1 (2725) | Full-length | SEQ ID NO:21 | SEQ ID NO:22 |
| EU-M-C terminus | BamH1 (2701) /Xho1 (2725) | 100-173 | SEQ ID NO:7 | SEQ ID NO:8 |
| VR-ORF7 | BamH1 (2701) /Xho1 (2725) | Full-length | SEQ ID NO:23 | SEQ ID NO:24 |
| VR-NSP7 | BamH1 (2701) /Xho1 (2725) | Full-length | SEQ ID NO:25 | SEQ ID NO:26 |
| VR-GP5-CE | BamH1 (2701) /Xho1 (2725) | aa 26-200 | SEQ ID NO:9 | SEQ ID NO:10 |
| VR-GP5-ecto | BamH1 (2701) /Xho1 (2725) | aa 26- 67 | SEQ ID NO:11 | SEQ ID NO:12 |
| VR-GP3-Mid | BamH1 (2701) /Xho1 (2725) | aa 61-201 | SEQ ID NO:13 | SEQ ID NO:14 |
| VR-M-C terminus | BamH1 (2701) /Xho1 (2725) | aa 97-174 | SEQ ID NO:15 | SEQ ID NO:16 |

The transformed cells were plated out on LB agar containing ampicillin (100 µg/ml) and cultivated over night at 37°C. On the following day, a single colony from the plate was resuspended in 5 ml of LB(Amp)-medium and incubated overnight at 37°C at 200 rpm. Subsequently, LB(Amp)-medium was inoculated with this culture in a ratio of 1:100. Again, the culture was incubated at 37°C and 200 rpm until an OD₆₀₀ of 0.6-0.8 was reached. At this point, the protein expression was induced by adding IPTG to a final concentration of 1 mM. Protein expression was allowed to take place over night at the above-mentioned conditions.

### EXAMPLE 2: Protein preparation and purification

The *E*. *coli* cultures described above were centrifuged for 20 min at 4000 × g. The supernatant was discarded and the pellet resuspended in lysis buffer (50 mM Tris pH 7.5, 2 mM EDTA) to a theoretical OD₆₀₀ of 50. The cells were sonicated on ice for 4 × 1 min and centrifuged afterwards (15 min at 10000 × g). The supernatant was dialyzed against a His-Tag binding buffer (20 mM Na₂HPO₄/NaH₂PO₄, pH 7.4, 500 mM NaCl, 20 mM Imidazole) at a ratio of 1:200.

This crude extract was subsequently loaded onto a GE Healthcare HisTrap column. Unbound protein was separated by a washing step using 10 column volumes (CV) of HisTag-binding buffer. In turn, the elution of the bound proteins was carried out using 10 CV of HisTag-Elution buffer (20 mM Na₂HPO₄/NaH₂PO₄, pH 7.4, 500 mM NaCl, 500 mM Imidazole). The chromatography run was analyzed via SDS-PAGE and fractions containing the desired protein were pooled and dialyzed over night against Maltose Binding Protein (MBP) binding buffer (20 mM Tris, pH 7.4, 200 mM NaCl, 1 mM EDTA) at a ratio of 1:200.

A column packed with Amylose Resin (New England Biolabs) was loaded with the dialyzed protein solution from the first purification step. Again, the unbound proteins were washed away with 5 CV MBP binding buffer. In the subsequent step the purified protein was eluted with 5 CV of MBP elution buffer (20 mM Tris, pH 7.4, 200 mM NaCl, 1 mM EDTA, 10 mM Maltose).

Finally, the protein identity and purity was confirmed via SDS-PAGE gel electrophoresis and the concentration of the purified proteins was determined by a BCA protein assay (Pierce).

### EXAMPLE 3: PRRSV ELISA utilizing expressed GP5 protein (for comparison reasons)

Two expressed GP5 protein fragments (SEQ ID NOs: 1 and 3) were used in an immunological detection of PRRSV antibodies and compared to expressed ORF7 protein (SEQ ID NO: 17). The ELISA was carried out with four sera in total. Two of the sera were known to be PRRSV positive, i.e. they were known to comprise PRRSV antibodies, and the remaining two sera were PRRSV negative, i.e. derived from a subject that had not been infected by PRRSV.

In a first step, the recombinant proteins and the negative control MBP are diluted to the appropriate concentration in 50 mM Na₂CO₃ buffer. The final concentration of PRRSV proteins and MBP was 20 ng/well. Other concentrations like 90 ng/well may also be used.

In a next step, 100 µl volumes of the diluted antigen are dispensed into an appropriate number of wells of a flat-bottomed, high protein binding ELISA microplate (Polysorp, Nunc 475094) using 2 wells per test serum as well as 2 wells for the negative control. Subsequently, the plate was sealed and incubated overnight at 4°C.

On the following day, the plates were washed 3 times with 1x PBS-Tween 0.05% and 200 µl blocking buffer (PBS with 5% low fat milk) was added. After sealing the plates were again incubated on an orbital shaker for 2 h at room temperature. Following incubation, the plates were washed 3 times with 1x PBS-Tween 0.05%.

In a next step, the sera were diluted 50 times in the blocking buffer and 100 µl were dispensed in each test well. Consequently, the plates were incubated on an orbital shake for 1 hour at room temperature and afterwards washed 3 times with 1x PBS-Tween 0.05%.

The working dilution of rabbit anti-pig immunoglobulin horse radish peroxidise conjugate in blocking buffer (1:10,000 Sigma A5670) was prepared and 100 µl were added to each test well. In turn, the plates were incubated on an orbital shaker for 1 hour at room temperature, washed as described before and immediately re-filled with 100 µl TMB One Solution (Promega G7431) as substrate. After incubation at room temperature for 30 min in a dark place without shaking, 50 µl of stopping solution (1 M sulphuric acid) were added.

The resulting absorbance values were measured on an ELISA reader at 450 nm and 520 nm as a reference.

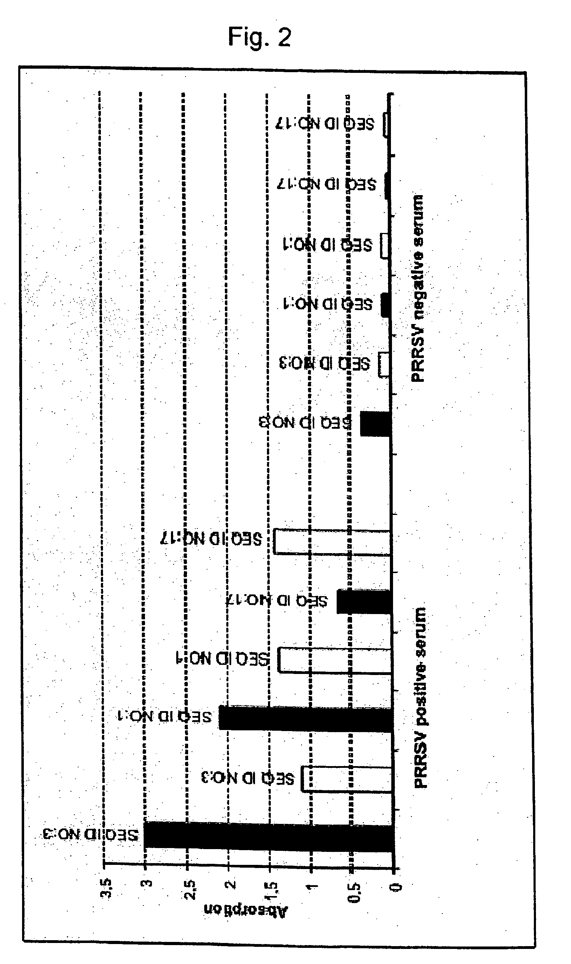

As can be observed from the results presented in FIG. 2, the recombinant GP5 proteins lead to improved antibody signals compared to the N protein (here, ORF7). The sensitivity of the immunological PRRSV detection is significantly increased, while the specificity is not negatively affected.

### EXAMPLE 4: PRRSV ELISA with multiple PRRSV proteins and comparison to IDEXX ELISA

A PRRSV ELISA utilizing multiple PRRSV antigens of the European strain was carried out. The proteins were individually coated on 96-well plates and analyzed in an ELISA using ten PRRSV-positive and negative sera each (N1-N10; P1-P10). The sera had previously been analyzed by the commercial available IDEXX ELISA to determine whether a given serum was PRRSV-positive or PRRSV-negative.

In a first step, the recombinant proteins and the negative control (MPB) were diluted to the appropriate concentration in 50 mM Na₂CO₃ buffer.

Afterwards, 100 µl volumes of the diluted antigen were dispensed into the wells of a flat-bottomed, high protein binding ELISA microplate (Polysorp, Nunc 475094) using 2 wells per test serum as well as 2 wells for the negative control. The final PRRSV-antigen concentrations used for the coating of the plates were: SEQ ID NO:17 20 ng/well; SEQ ID NO:19 15 ng/well; SEQ ID NO:1 20 ng/well; and SEQ ID NO:7 100 ng/well. The plate was sealed and incubated overnight at 4°C.

On the following day, the plates were washed 3 times with 1x PBS-Tween 0.05% and 200 µl blocking buffer (PBS with 5% low fat milk) was added. After sealing, the plates were incubated on an orbital shaker for 2 h at room temperature and subsequently washed 3 times with 1x PBS-Tween 0.05%.

The sera were diluted 50 times in blocking buffer and 100 µl were dispensed in each test well. The plates were then incubated on an orbital shaker for 1 hour at room temperature and washed 3 times with 1x PBS-Tween 0.05%.

The working dilution of rabbit anti-pig immunoglobulin horse radish peroxidise conjugate (1:10,000 Sigma A5670) was prepared in blocking buffer and 100 µl were added to each test well. In turn, the plates were incubated on an orbital shaker for 1 hour at room temperature.

Following this period, the plates were washed as before and immediately re-filled with 100 µl TMB One Solution (Promega G7431) as a substrate and incubated at room temperature for 30 min in a dark environment without shaking. 50 µl of stopping solution consisting of 1 M sulphuric acid was then added.

The resulting absorbance values are measured on an ELISA reader at 450 nm and 520 nm as a reference.

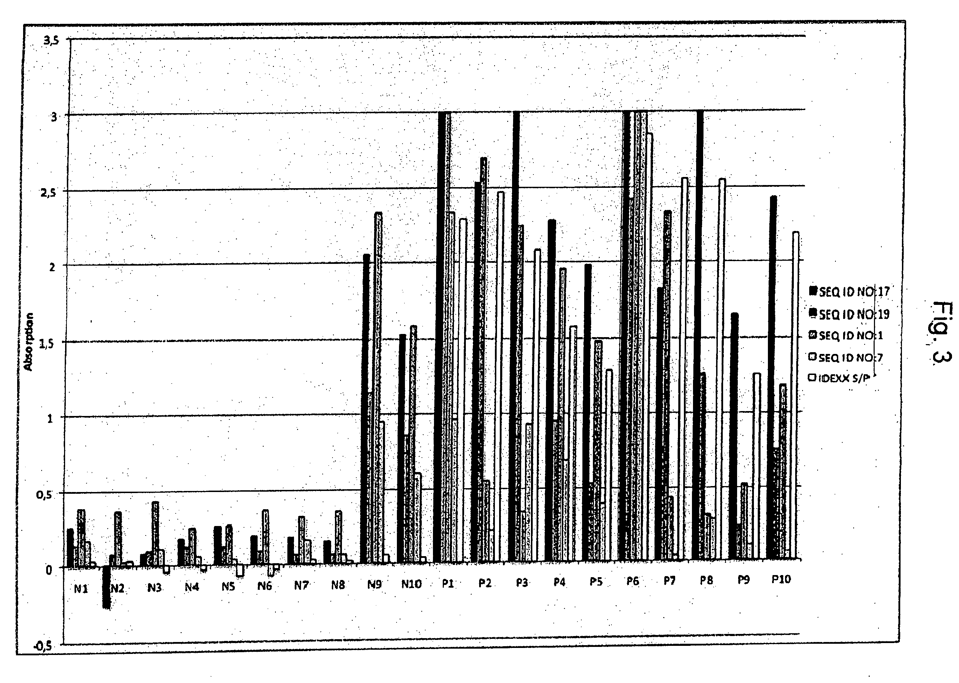

As can be observed from the results depicted in FIG. 3, an ELISA assay combining multiple PRRSV proteins, according to the invention, yields markedly more specific and sensitive PRRSV antibody detection then the commercially available IDEXX ELISA. All of the samples P1-P10 tested positive and likewise tested positive using the ELISA assay according to the invention, and partially with even higher absorption readings. Notably, of the ten samples, N1-N10 tested negative using the IDEXX ELISA, yet samples N9 and N10 were clearly positive in the ELISA assay according to the invention. These results were further confirmed by an ELISA assay utilizing SEQ ID NO: 3 and/or SEQ ID NO: 21 as a PRRSV antigen (for comparison reasons).

To summarize, the ELISA assay provided by the present invention provided for the detection of two PRRSV positive sera that were tested as false-negatives using the commercially available IDEXX ELISA assay.

### EXAMPLE 5: PRRSV ELISA with PRRSV proteins of a North American strain

The ELISA assays described in Examples 3 and 4 herein were repeated with the corresponding PRRSV proteins of a North American strain. Thus, SEQ ID NOs: 1, 3, and 17 of Example 3 were exchanged with SEQ ID NOs: 9, 11, and 23 (for comparison reasons). Furthermore, SEQ ID NOs: 17, 19, 1, and 7 of Example 4 were exchanged with SEQ ID NOs: 23, 25, 9, 15.

The ELISA assay according to the present invention utilizing PRRSV proteins from a North American strain were shown to yield similar results as those obtained using the European strain as described above (Lelystad Virus (LV)).

### EXAMPLE 6: PRRSV ELISA with the three PRRSV proteins GP5, NSp7 and GP3

ELISA assays employing various combinations of the PRRSV proteins, GP5, NSp7 and GP3 were tested. The ELISA assays were carried out as described in Examples 3-5 above.

The NSp7 protein used was SEQ ID NO: 19. The GP5 protein used was either the GP5 fragment set forth in SEQ ID NO: 1 or SEQ ID NO: 3. The GP3 protein used was the GP3 fragment set forth in SEQ ID NO: 5.

Concentration of each individual protein was 30 ng/well, hence 90 in total ng/well.

While all of the above combinations were observed to work extremely well, a combination of SEQ ID NO 19, SEQ ID NO: 1 and SEQ ID NO: 5 yielded the best, most effective results.

The ELISA assays described above were repeated with the corresponding PRRSV proteins of a North American strain and yielded similar results. Thus, SEQ ID NOs: 19, 1, and 3 were exchanged for SEQ ID NOs: 25, 9 and 11.

### EXAMPLE 7: Comparison of an ELISA according to an embodiment of the present invention with the commercially available PRRSV-ELISA systems IDEXX. INGENASA and Hipra

To compare the performance of a PRRSV ELISA according to a preferred embodiment of the present invention with other available assays, eighteen porcine serum samples from a proficiency testing scheme (PTS) for PRRSV were analyzed with three different commercially available PRRSV-ELISAs (IDEXX, INGENASA and Hipra) and with an ELISA comprising antigens of the SEQ IDs 1 and 9 (GP5), SEQ IDs 5 and 13 (GP3) and SEQ IDs 19 and 25 (NSP7). The samples had a defined infection status, for six of them with a defined PRRSV genotype.

The ELISA assays were carried out as described in Examples 3-6 above or according to the manufacturer's instructions.

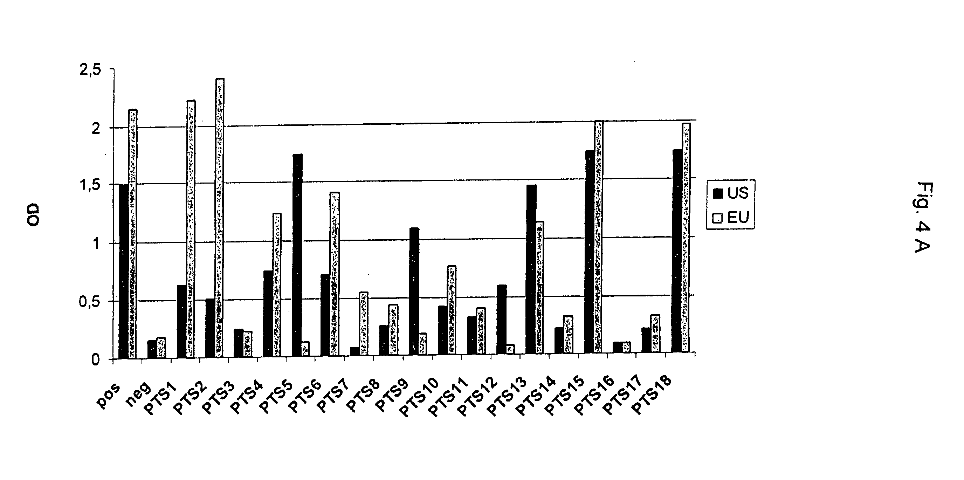

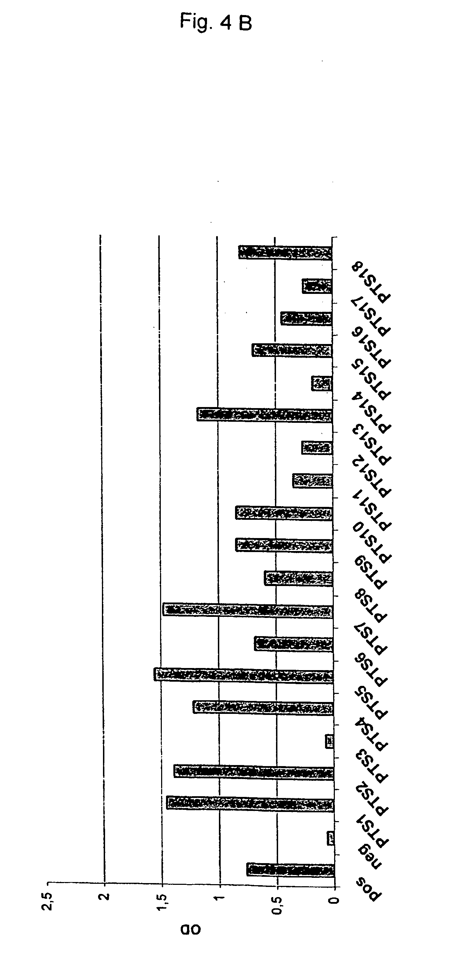

FIG. 4A illustrates the optical density-results for the PRRSV ELISA comprising antigens of the SEQ IDs 1 and 9 (GP5), SEQ IDs 5 and 13 (GP3) and SEQ IDs 19 and 25 (NSP7). The optical density-results are depicted separately for US and EU antigens. FIG. 4B depicts the optical density-results obtained for the IDEXX HerdCheck PRRS X3 ELISA. Cut-Off values were calculated according to the manufacturer's instructions or by adding three standard deviations to the internal negative control (neg) for the PRRSV ELISA according to the present invention. For INGENASA and HIPRA, results were obtained from the final report of the PTS trial (Wellenberg et al., GD BV Animal Health Service Ltd., Deventer, The Netherlands, 2010)

All test systems failed to detect antibodies in the negative pig serum (sample PTS3, FIG. 4, Table 2). However, the PRRSV ELISA according to a preferred embodiment of the present invention performed best in correctly recognizing the other sera (Table 2). It was the most sensitive assay, as it detected the highly diluted samples 14 and 17. Two samples (PTS12 and PTS14) were only detected as positive by the PRRSV ELISA according to an embodiment of the present invention. Furthermore, the PRRSV ELISA according to an embodiment of the present invention was most successful in distinguishing PRRSV infections with American from infections with the European genotype (Table 3). For instance, antibodies present in samples PTS5 and PTS12 (both infected with an US genotype virus) only recognized the US antigen-mix.

The three commercially available ELISAs contain a single antigen, whereas the PRRSV ELISA according to an embodiment of the present invention consists of three different antigens. The data presented here clearly show that there is a significant advantage of the present invention in sensitivity and differentiation of genotypes compared with other, established systems.

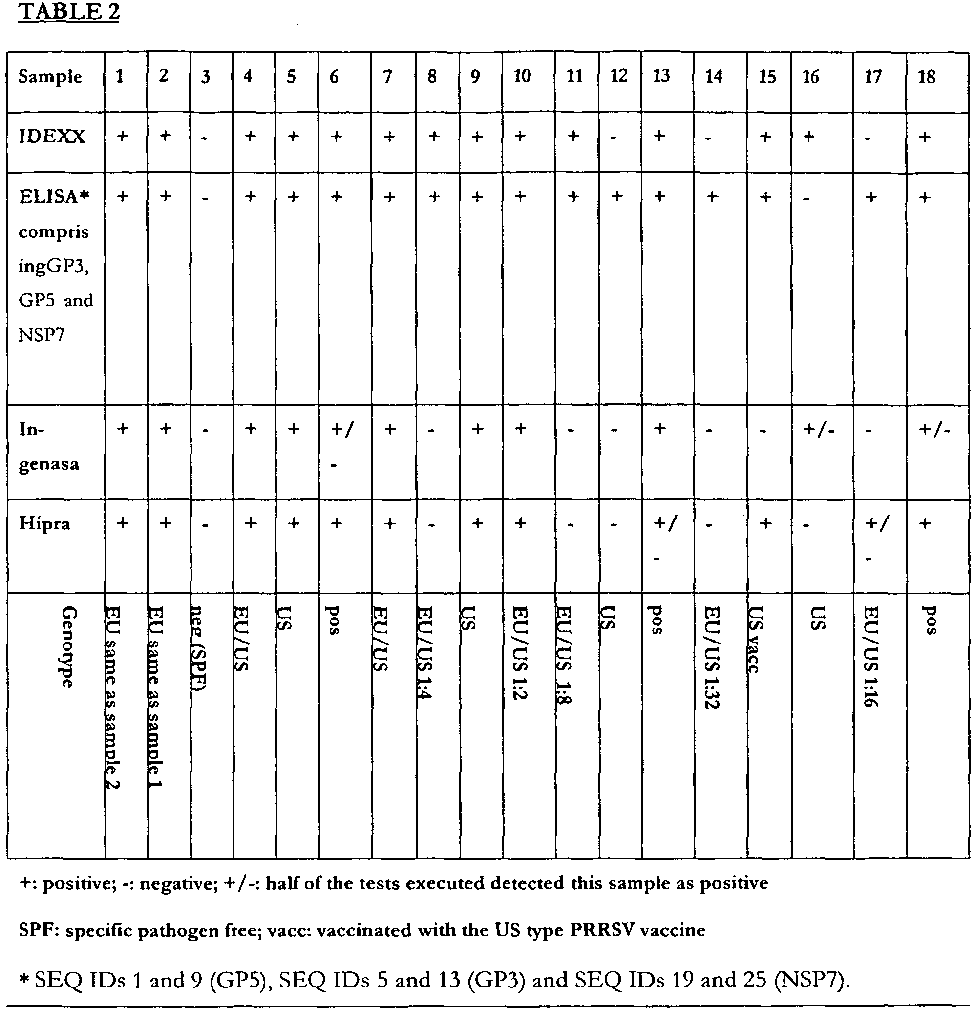

**TABLE 3**

| **ID** | **Genotype** | **EU/US differentiated by** |
|---|---|---|
| 1 | EU | Ingenasa (2.5 x more OD * |
| | | PRRSV ELISA according to the present invention (4 x more OD) |
| 2 | EU | Ingenasa (2.5 x more OD)* |
| | | ELISA** comprisingGP3, GP5 and NSP7 (4 x more OD)* |
| 5 | US | ELISA** comprisingGP3, GP5 and NSP7 |
| 9 | US | Ingenasa (50%), Hipra |
| | | ELISA** comprisingGP3, GP5 and NSP7 |
| 12 | US | ELISA** comprisingGP3, GP5 and NSP7 |
| 15 | US | Hipra (2-3 x more OD)* |
| 16 | US | Ingenasa (50%) |
| * OD-value of the test specific for one genotype compared to the test specific for the other genotype | | |
| ** SEQ IDs 1 and 9 (GP5), SEQ IDs 5 and 13 (GP3) and SEQ IDs 19 and 25 (NSP7). | | |

### EXAMPLE 8: Synergistic effects between the single antigens of PRRSV ELISA

It is important to test which antigens comprised in the test are the critical ones. These are the antigens eliciting the highest antibody signal. Unexpectedly, a clear synergistic effect is seen with a combination of the individual antigens is analysed in a PRRSV ELISA according to a preferred embodiment of the present invention, comprising antigens of the SEQ IDs 1 and 9 (GP5), SEQ IDs 5 and 13 (GP3) and SEQ IDs 19 and 25 (NSP7). The ELISA assay was carried out as described in Examples 3-7 above.

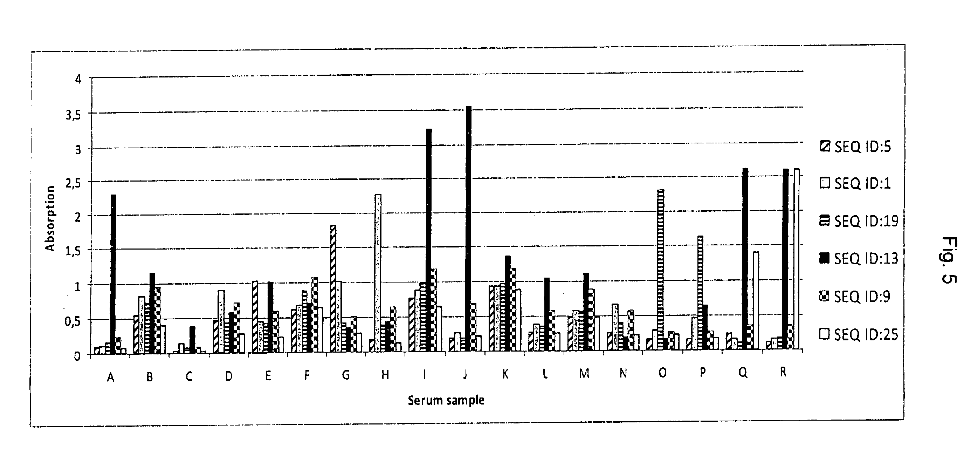

FIG. 5 shows 18 different PRRSV-positive pig sera, reacting with the individual antigens present in the PRRSV ELISA antigen mixture. The antigens that elicit the strongest signal vary significandy between the serum samples. In some sera, NSP 7 (SEQ ID NO:19 and 25) is the dominant antigen. Other sera recognize GP3 (SEQ ID NO:5 and 13) or GP5 (SEQ IDNO:1 and 9) most strongly. Therefore, to detect all the sera as positive, the presence of all three of the antigens is essential.

As examples, sera A and C are only detected as positive because antibodies against GP3 (SEQ ID NO:13) are present, similarly to NSP7 (SEQ ID NO:19) in serum O. In serum G, the dominant antibody response is measured against GP3 (SEQ ID NO:5) and GP5 (SEQ ID NO:1). In other examples (L), the presence of two antigens allows for the discrimination between infections by the US and EU-strains. Here, using only US_GP5 (SEQ ID NO:9) would lead to a difference which would be far less significant compared to the addition of the signals elicited by US_GP3 (SEQ ID NO:13). In sera Q and R, addition of the GP3 antigen (SEQ ID NO: 13) to the NSP7 antigen (SEQ ID NO:25) leads to a significantly increased antibody signal and enables a clearer discrimination of the PRRSV US genotype against the EU genotype.

Hence, due to the high degree of variability in the anti-PRRSV antibody responses of pigs, a clear synergistic effect is seen by using the mixture of antigens, namely NSP7, GP5 and GP3 of both genotypes. The presence of all three of these antigens is essential to detect the maximum numbers of PRRSV infections. This leads to a significant advantage over available technologies relying only on one antigen or on antigen mixtures different from the one used according to a preferred embodiment of the present invention.

In the claims, the word "comprising" does not exclude other elements or steps, and the indefinite article "a" or "an" does not exclude a plurality.

## Claims

1. A method for the detection of porcine reproductive and respiratory syndrome virus (PRRSV) antibodies comprising the steps of:
i. providing at least two PRRSV proteins, wherein one of the at least two PRRSV proteins is the GP5 protein, and wherein one of the at least two PRRSV proteins is the NSp7 protein, or fragments thereof that are recognized as an epitope by a PRRSV antibody and have a length of at least 10 continuous amino acids;
ii. contacting the at least two PRRSV proteins or fragments thereof with a liquid sample that is derivable from an individual to be tested under conditions to allow the binding of PRRSV antibodies in the liquid sample that is derivable from an individual to be tested to the PRRSV proteins or fragments; and
iii. detecting the PRRSV antibodies bound to the PRRSV proteins or fragments thereof.

2. The method according to claim 1, wherein the at least two PRRSV proteins further comprise a protein selected from the group consisting of GP3, M-protein, E-protein, and N-protein.

3. The method according to any of the preceding claims, wherein at least one of the PRRSV proteins is a recombinant protein.

4. The method according to claim 3, wherein at least one of the PRRSV proteins further comprises an MBP-tag and/or a His-tag.

5. The method according to any of the preceding claims, wherein at least one of the PRRSV proteins is derived from a European PRRSV strain Lelystad or a North American PRRSV strain, preferably VR-2332.

6. The method according to any of the preceding claims, wherein the PRRSV proteins or fragments thereof provided are a fragment of the GP5 protein, preferably SEQ ID NO: 1 or 9, the NSp7 protein, preferably SEQ ID NO: 19 or 25, and a fragment of the GP3 protein, preferably SEQ ID NO: 5 or 13.

7. The method according to any of claims 1-5, wherein the PRRSV proteins or fragments thereof provided comprise the proteins or fragments thereof with SEQ ID NOs: 1, 9, 19, 25, 5 and 13.

8. The method according to any of the preceding claims, wherein the liquid sample that is derivable from an individual to be tested is selected from the group consisting of blood, serum, sputum, saliva and/or milk.

9. The method according to any of the preceding claims, wherein the detection is carried out immunologically.

10. The method according to any of the preceding claims, wherein the detection of PRRSV antibodies indicates a PRRSV infection or an effective PRRSV vaccination.

11. A system for the detection of PRRSV antibodies comprising a support and immobilized thereon at least two PRRSV proteins, wherein one of the at least two PRRSV proteins is the GP5 protein, and wherein one of the at least two PRRSV proteins is the NSp7 protein, or fragments thereof that are recognized as an epitope by a PRRSV antibody and have a length of at least 10 continuous amino acids.

12. A kit comprising at least two PRRSV proteins, wherein one of the at least two PRRSV proteins is the GP5 protein, and wherein one of the at least two PRRSV proteins is the NSp7 protein, or fragments thereof that are recognized as an epitope by a PRRSV antibody and have a length of at least 10 continuous amino acids.

13. The system according to claim 11 or the kit according to claim 12, wherein the proteins or fragments thereof provided are a fragment of the GP5 protein, preferably SEQ ID NO: 1 or 9, the NSp7 protein, preferably SEQ ID NO: 19 or 25, and a fragment of the GP3 protein, preferably SEQ ID NO: 5 or 13.

14. The system according to claim 11 or the kit according to claim 12, the system or kit comprising the PRRSV proteins or fragments thereof with SEQ ID NOs: 1, 9, 19, 25, 5 and 13.

## Patentansprüche

1. Ein Verfahren zum Nachweis von Antikörpern gegen porcines Reproduktions- und Replikationssyndrom-Virus (PRRSV) umfassend die Schritte:
i. Bereitstellen von mindestens zwei PRRSV-Proteinen, wobei eines der mindestens zwei PRRSV-Proteine das GP5-Protein ist, und wobei eines der mindestens zwei PRRSV-Proteine das NSp7-Protein ist, oder Fragmenten davon, die von einem PRRSV-Antikörper als ein Epitop erkannt werden und eine Länge von mindestens 10 zusammenhängenden Aminosäuren haben;
ii. Kontaktieren der mindestens zwei PRRSV-Proteine oder Fragmente davon mit einer flüssigen Probe, die von einem zu testenden Individuum ableitbar ist, unter Bedingungen, um die Bindung von PRRSV-Antikörpern in der flüssigen Probe, die von einem zu testenden Individuum ableitbar ist, an die PRRSV-Proteine oder Fragmente zu ermöglichen; und
iii. Nachweisen der an die PRRSV-Proteine oder Fragmente davon gebundenen PRRSV-Antikörper.

2. Das Verfahren gemäß Anspruch 1, wobei die mindestens zwei PRRSV-Proteine weiter ein Protein ausgewählt aus der Gruppe bestehend aus GP3, M-Protein, E-Protein und N-Protein umfassen.

3. Das Verfahren gemäß irgendeinem der vorhergehenden Ansprüche, wobei mindestens eines der PRRSV-Proteine ein rekombinantes Protein ist.

4. Das Verfahren gemäß Anspruch 3, wobei mindestens eines der PRRSV-Proteine weiter ein MBP-Anhängsel und/oder ein His-Anhängsel umfasst.

5. Das Verfahren gemäß irgendeinem der vorhergehenden Ansprüche, wobei mindestens eines der PRRSV-Proteine von einem europäischen PRRSV-Stamm Lelystad oder von einem nordamerikanischen PRRSV-Stamm, vorzugsweise VR-2332, abgeleitet ist.

6. Das Verfahren gemäß irgendeinem der vorhergehenden Ansprüche, wobei die bereitgestellten PRRSV-Proteine oder Fragmente davon ein Fragment des GP5-Proteins, vorzugsweise SEQ ID NO: 1 oder 9, das NSp7-Protein, vorzugsweise SEQ ID NO: 19 oder 25, und ein Fragment des GP3-Proteins, vorzugsweise SEQ ID NO: 5 oder 13, sind.

7. Das Verfahren gemäß irgendeinem der Ansprüche 1-5, wobei die bereitgestellten PRRSV-Proteine oder Fragmente davon die Proteine oder Fragmente davon mit SEQ ID NOs: 1, 9, 19, 25, 5 und 13 umfassen.

8. Das Verfahren gemäß irgendeinem der vorhergehenden Ansprüche, wobei die flüssige Probe, die von einem zu testenden Individuum ableitbar ist, ausgewählt ist aus der Gruppe bestehend aus Blut, Serum, Auswurf, Speichel und/oder Milch.

9. Das Verfahren gemäß irgendeinem der vorhergehenden Ansprüche, wobei der Nachweis immunologisch durchgeführt wird.

10. Das Verfahren gemäß irgendeinem der vorhergehenden Ansprüche, wobei der Nachweis von PRRSV-Antikörpern eine PRRSV-Infektion oder eine wirksame PRRSV-Impfung anzeigt.

11. Ein System zum Nachweis von PRRSV-Antikörpern umfassend einen Träger und immobilisiert darauf mindestens zwei PRRSV-Proteine, wobei eines der mindestens zwei PRRSV-Proteine das GP5-Protein ist, und wobei eines der mindestens zwei PRRSV-Proteine das NSp7-Protein ist, oder Fragmente davon, die von einem PRRSV-Antikörper als ein Epitop erkannt werden und eine Länge von mindestens 10 zusammenhängenden Aminosäuren haben.

12. Ein Kit umfassend mindestens zwei PRRSV-Proteine, wobei eines der mindestens zwei PRRSV-Proteine das GP5-Protein ist, und wobei eines der mindestens zwei PRRSV-Proteine das NSp7-Protein ist, oder Fragmente davon, die von einem PRRSV-Antikörper als ein Epitop erkannt werden und eine Länge von mindestens 10 zusammenhängenden Aminosäuren haben.

13. Das System gemäß Anspruch 11 oder der Kit gemäß Anspruch 12, wobei die bereitgestellten PRRSV-Proteine oder Fragmente davon ein Fragment des GP5-Proteins, vorzugsweise SEQ ID NO: 1 oder 9, das NSp7-Protein, vorzugsweise SEQ ID NO: 19 oder 25, und ein Fragment des GP3-Proteins, vorzugsweise SEQ ID NO: 5 oder 13, sind.

14. Das System gemäß Anspruch 11 oder der Kit gemäß Anspruch 12, das System oder der Kit umfassend die PRRSV-Proteine oder Fragmente davon mit SEQ ID NOs: 1, 9, 19, 25, 5 und 13.

## Revendications

1. Procédé de détection d'anticorps dirigés contre le virus du syndrome dysgénésique et respiratoire porcin (PRRSV) comprenant les étapes suivantes :
i. la fourniture d'au moins deux protéines du PRRSV, dans lequel l'une des au moins deux protéines du PRRSV est la protéine GP5, et dans lequel l'une des au moins deux protéines du PRRSV est la protéine NSp7, ou des fragments de celles-ci qui sont reconnus comme un épitope par un anticorps anti-PRRSV et ont une longueur d'au moins 10 acides aminés consécutifs ;
ii. la mise en contact des au moins deux protéines du PRRSV ou de fragments de celles-ci avec un échantillon de liquide qui peut être obtenu d'un individu à tester dans des conditions pour permettre la liaison des anticorps anti-PRRSV dans l'échantillon de liquide qui peut être obtenu d'un individu à tester aux protéines du PRRSV ou aux fragments ; et
iii. la détection des anticorps anti-PRRSV liés aux protéines du PRRSV ou à des fragments de celles-ci.

2. Procédé selon la revendication 1, dans lequel les au moins deux protéines du PRRSV comprennent en outre une protéine choisie dans le groupe constitué de GP3, protéine M, protéine E et protéine N.

3. Procédé selon l'une quelconque des revendications précédentes, dans lequel au moins l'une des protéines du PRRSV est une protéine recombinante.

4. Procédé selon la revendication 3, dans lequel au moins l'une des protéines du PRRSV comprend en outre un marqueur MBP et/ou un marqueur His.

5. Procédé selon l'une quelconque des revendications précédentes, dans lequel au moins l'une des protéines du PRRSV est obtenue d'une souche européenne du PRRSV Lelystad ou d'une souche nord-américaine du PRRSV, de préférence VR-2332.

6. Procédé selon l'une quelconque des revendications précédentes, dans lequel les protéines du PRRSV ou les fragments de celles-ci fournis sont un fragment de la protéine GP5, de préférence SEQ ID NO : 1 ou 9, la protéine NSp7, de préférence SEQ ID NO : 19 ou 25, et un fragment de la protéine GP3, de préférence SEQ ID NO : 5 ou 13.

7. Procédé selon l'une quelconque des revendications 1 à 5, dans lequel les protéines du PRRSV ou les fragments de celles-ci fournis comprennent les protéines ou des fragments de celles-ci avec SEQ ID NO : 1, 9, 19, 25, 5 et 13.

8. Procédé selon l'une quelconque des revendications précédentes, dans lequel l'échantillon de liquide qui peut être obtenu d'un individu à tester est choisi dans le groupe constitué de sang, sérum, crachats, salive et/ou lait.

9. Procédé selon l'une quelconque des revendications précédentes, dans lequel la détection est réalisée par immunologie.

10. Procédé selon l'une quelconque des revendications précédentes, dans lequel la détection d'anticorps anti-PRRSV indique une infection par le PRRSV ou une vaccination efficace contre le PRRSV.

11. Système de détection d'anticorps anti-PRRSV comprenant un support et, immobilisées dessus, au moins deux protéines du PRRSV, dans lequel l'une des au moins deux protéines du PRRSV est la protéine GP5, et dans lequel l'une des au moins deux protéines du PRRSV est la protéine NSp7, ou des fragments de celles-ci qui sont reconnus comme un épitope par un anticorps anti-PRRSV et ont une longueur d'au moins 10 acides aminés consécutifs.

12. Kit comprenant au moins deux protéines du PRRSV, dans lequel l'une des au moins deux protéines du PRRSV est la protéine GP5, et dans lequel l'une des au moins deux protéines du PRRSV est la protéine NSp7, ou des fragments de celles-ci qui sont reconnus comme un épitope par un anticorps anti-PRRSV et ont une longueur d'au moins 10 acides aminés consécutifs.

13. Système selon la revendication 11 ou kit selon la revendication 12, dans lequel les protéines ou les fragments de celles-ci fournis sont un fragment de la protéine GP5, de préférence SEQ ID NO : 1 ou 9, la protéine NSp7, de préférence SEQ ID NO : 19 ou 25, et un fragment de la protéine GP3, de préférence SEQ ID NO : 5 ou 13.

14. Système selon la revendication 11 ou kit selon la revendication 12, le système ou le kit comprenant les protéines du PRRSV ou des fragments de celles-ci avec SEQ ID NO : 1, 9, 19, 25, 5 et 13.


### SEQUENCE LISTING

<110> Fraunhofer-Gesellschaft zur Förderung der angewandten Forschung e.V.
<120> Methods for PRRSV Detection
<130> 144-012P
<150> 09 012 930.5
   <151> 2009-10-13
<160> 28
<170> PatentIn version 3.3
<210> 1
   <211> 168
   <212> PRT
   <213> Artificial
<220>
   <223> aa 33-201 of the GP5 protein of the the European strain of PRRSV (Lelystad Virus)
<400> 1
   Gly Asn Gly Asn Ser Ser Thr Tyr Gln Tyr Ile Tyr Asn Leu Thr Ile
   1               5                   10                  15
   Cys Glu Leu Asn Gly Thr Asp Trp Leu Ser Ser His Phe Gly Trp Ala
               20                  25                  30
   Val Glu Thr Phe Val Phe Tyr Pro Val Ala Thr His Ile Leu Ser Leu
           35                  40                  45
   Gly Phe Leu Thr Thr Ser His Phe Phe Asp Ala Leu Gly Leu Gly Ala
       50                  55                  60
   Val Ser Thr Ala Gly Phe Val Gly Gly Arg Tyr Val Leu Cys Ser Val
   65                  70                  75                  80
   Tyr Gly Ala Cys Ala Phe Ala Ala Phe Val Cys Phe Val Ile Arg Ala
                   85                  90                  95
   Ala Lys Asn Cys Met Ala Cys Arg Tyr Ala Arg Thr Arg Phe Thr Asn
               100                 105                 110
   Phe Ile Val Asp Asn Arg Gly Arg Val His Arg Trp Lys Ser Pro Ile
           115                 120                 125
   Val Val Glu Lys Leu Gly Lys Ala Glu Val Asp Gly Asn Leu Val Thr
       130                 135                 140
   Ile Lys His Val Val Leu Glu Gly Val Lys Ala Gln Pro Leu Thr Arg
   145                 150                 155                 160
   Thr Ser Ala Glu Gln Trp Glu Ala
                   165
<210> 2
   <211> 504
   <212> DNA
   <213> artificial
<220>
   <223> cDNA for the expression of aa 33-201 of the GP5 protein of the the European strain of PRRSV (Lelystad Virus)
<400> 2
   ggcaacggca acagctcgac ataccaatac atatataact tgacgatatg cgagctgaat      60
   gggaccgact ggttgtccag ccattttggt tgggcagtcg agacctttgt gttttacccg     120
   gttgccactc atatcctctc actgggtttt ctcacaacaa gccatttttt tgacgcgctc     180
   ggtctcggcg ctgtatccac tgcaggattt gttggcgggc ggtatgtact ttgcagcgtc     240
   tacggcgctt gtgctttcgc agcgttcgta tgttttgtca tccgtgctgc taaaaattgc     300
   atggcctgcc gctatgcccg tacccggttt accaacttca ttgtagacaa ccgggggaga     360
   gttcatcgat ggaagtctcc aatagtggta gaaaaattgg gcaaagccga agtcgacggc     420
   aacctcgtca ccatcaaaca tgtcgtcctc gaaggggtta aagctcaacc cttgacgagg     480
   acttcggctg agcaatggga ggcc                                            504
<210> 3
   <211> 32
   <212> PRT
   <213> artificial
<220>
   <223> aa 33-66 of the GP5 protein of the the European strain of PRRSV (Lelystad Virus)
<400> 3
   Gly Asn Gly Asn Ser Ser Thr Tyr Gln Tyr Ile Tyr Asn Leu Thr Ile
   1               5                   10                  15
   Cys Glu Leu Asn Gly Thr Asp Trp Leu Ser Ser His Phe Gly Trp Ala
               20                  25                  30
<210> 4
   <211> 96
   <212> DNA
   <213> artificial
<220>
   <223> cDNA for the expression of aa 33-66 of the GP5 protein of the the European strain of PRRSV (Lelystad Virus)
<400> 4
   ggcaacggca acagctcgac ataccaatac atatataact tgacgatatg cgagctgaat      60
   gggaccgact ggttgtccag ccattttggt tgggca                                96
<210> 5
   <211> 184
   <212> PRT
   <213> artificial
<220>
   <223> aa 55-238 of the GP3 protein of the the European strain of PRRSV (Lelystad Virus)
<400> 5
   Met Pro Cys Ser Thr Ser Gln Ala Ala Arg Gln Arg Leu Glu Pro Gly
   1               5                   10                  15
   Arg Asn Met Trp Cys Lys Ile Gly Tyr Asp Arg Cys Glu Glu Arg Asp
               20                  25                  30
   His Asp Glu Leu Leu Met Pro Ile Pro Ser Gly Tyr Asp Asn Leu Lys
           35                  40                  45
   Leu Glu Gly Tyr Tyr Ala Trp Leu Ala Phe Leu Ser Phe Ser Tyr Ala
       50                  55                  60
   Ala Gln Phe His Pro Glu Leu Phe Gly Ile Gly Asn Val Ser Arg Val
   65                  70                  75                  80
   Phe Val Asp Lys Arg His Gln Phe Ile Cys Ala Glu His Asp Gly Gln
                   85                  90                  95
   Asn Ser Thr Val Ser Thr Gly His Asn Ile Ser Ala Leu Tyr Ala Ala
               100                 105                 110
   Tyr Tyr His His Gln Ile Asp Gly Gly Asn Trp Phe His Leu Glu Trp
           115                 120                 125
   Leu Arg Pro Leu Phe Ser Ser Trp Leu Val Leu Asn Ile Ser Trp Phe
       130                 135                 140
   Leu Arg Arg Ser Pro Val Ser Pro Val Ser Arg Arg Ile Tyr Gln Ile
   145                 150                 155                 160
   Leu Arg Pro Thr Arg Pro Arg Leu Pro Val Ser Trp Ser Phe Arg Thr
                   165                 170                 175
   Ser Ile Val Ser Asp Leu Thr Gly
               180
<210> 6
   <211> 552
   <212> DNA
   <213> artificial
<220>
   <223> cDNA for the expression of aa 55-238 of the GP3 protein of the the European strain of PRRSV (Lelystad Virus)
<400> 6
   atgccctgtt ctaccagtca agcggctcgc caaaggctcg agcccggtcg taacatgtgg     60
   tgcaaaatag ggtatgacag gtgtgaggag cgtgaccatg atgagttgtt aatgcccatc    120
   ccgtccgggt acgacaacct caaacttgag ggttattatg cttggctggc ttttttgtcc     180
   ttttcctacg cggcccaatt ccatccggag ttgttcggga tagggaatgt gtcgcgcgtc    240
   ttcgtggaca agcgacacca gttcatttgt gccgagcatg atggacagaa ttcaaccgta    300
   tctaccggac acaacatctc cgcattatat gcggcatatt accaccacca aatagacggg    360
   ggcaattggt tccatttgga atggctgcgg ccactctttt cctcctggct ggtgctcaat    420
   atatcatggt ttttgaggcg ttcgcctgta agccctgttt ctcgacgcat ctatcagata    480
   ttgagaccaa cacgaccgcg gctgccggtt tcatggtcct tcaggacatc aattgtctcc    540
   gacctcacgg gg                                                        552
<210> 7
   <211> 74
   <212> PRT
   <213> artificial
<220>
   <223> aa 100-173 of the M protein of the the European strain of PRRSV (Lelystad Virus)
<400> 7
   Ala Cys Cys Leu Gly Arg Arg Tyr Ile Leu Ala Pro Ala His His Val
   1               5                   10                  15
   Glu Ser Ala Ala Gly Leu His Ser Ile Ser Ala Ser Gly Asn Arg Ala
               20                  25                  30
   Tyr Ala Val Arg Lys Pro Gly Leu Thr Ser Val Asn Gly Thr Leu Val
           35                  40                  45
   Pro Gly Leu Arg Ser Leu Val Leu Gly Gly Lys Arg Ala Val Lys Arg
       50                  55                  60
   Gly Val Val Asn Leu Val Lys Tyr Gly Arg
   65                  70
<210> 8
   <211> 222
   <212> DNA
   <213> artificial
<220>
   <223> cDNA for the expression of aa 100-173 of the M protein of the the European strain of PRRSV (Lelystad Virus)
<400> 8
   gcatgttgcc ttggccggcg atacattctg gcccctgccc atcacgtaga aagtgctgca     60
   ggtctccatt caatctcagc gtctggtaac cgagcatacg ctgtgagaaa gcccggacta    120
   acatcagtga acggcactct agtaccagga cttcggagcc tcgtgctggg cggcaaacga    180
   gctgttaaac gaggagtggt taacctcgtc aagtatggcc gg                       222
<210> 9
   <211> 175
   <212> PRT
   <213> artificial
<220>
   <223> aa 66-200 of the GP5 protein of the the North American PRRSV strain VR-2332
<400> 9
   Ala Val Leu Ala Asn Ala Ser Asn Asp Ser Ser Ser His Leu Gln Leu
   1               5                   10                  15
   Ile Tyr Asn Leu Thr Leu Cys Glu Leu Asn Gly Thr Asp Trp Leu Ala
               20                  25                  30
   Asn Lys Phe Asp Trp Ala Val Glu Ser Phe Val Ile Phe Pro Val Leu
           35                  40                  45
   Thr His Ile Val Ser Tyr Gly Ala Leu Thr Thr Ser His Phe Leu Asp
       50                  55                  60
   Thr Val Ala Leu Val Thr Val Ser Thr Ala Gly Phe Val His Gly Arg
   65                  70                  75                  80
   Tyr Val Leu Ser Ser Ile Tyr Ala Val Cys Ala Leu Ala Ala Leu Thr
   85 90 95
   Cys Phe Val Ile Arg Phe Ala Lys Asn Cys Met Ser Trp Arg Tyr Ala
   100 105 110
   Cys Thr Arg Tyr Thr Asn Phe Leu Leu Asp Thr Lys Gly Arg Leu Tyr
   115 120 125
   Arg Trp Arg Ser Pro Val Ile Ile Glu Lys Arg Gly Lys Val Glu Val
   130 135 140
   Glu Gly His Leu Ile Asp Leu Lys Arg Val Val Leu Asp Gly Ser Val
   145 150 155 160
   Ala Thr Pro Ile Thr Arg Val Ser Ala Glu Gln Trp Gly Arg Pro
   165 170 175
<210> 10
   <211> 525
   <212> DNA
   <213> artificial
<220>
   <223> cDNA for the expression of aa 66-200 of the GP5 protein of the the North American PRRSV strain VR-2332
<400> 10
   gctgtgctcg ccaacgccag caacgacagc agctcccatc tacagctgat ttacaacttg 60
   acgctatgtg agctgaatgg cacagattgg ctagctaaca aatttgattg ggcagtggag 120
   agttttgtca tctttcccgt tttgactcac attgtctcct atggtgccct cactaccagc 180
   catttccttg acacagtcgc tttagtcact gtgtctaccg ccgggtttgt tcacgggcgg 240
   tatgtcctaa gtagcatcta cgcggtctgt gccctggctg cgttgacttg cttcgtcatt 300
   aggtttgcaa agaattgcat gtcctggcgc tacgcgtgta ccagatatac caactttctt 360
   ctggacacta agggcagact ctatcgttgg cggtcgcctg tcatcataga gaaaaggggc 420
   aaagttgagg tcgaaggtca tctgatcgac ctcaaaagag ttgtgcttga tggttccgtg 480
   gcaaccccta taaccagagt ttcagcggaa caatggggtc gtcct 525
<210> 11
   <211> 41
   <212> PRT
   <213> artificial
<220>
   <223> aa 26-67 of the GP5 protein of the the North American PRRSV strain VR-2332
<400> 11
   Ala Val Leu Ala Asn Ala Ser Asn Asp Ser Ser Ser His Leu Gln Leu
   1               5                   10                  15
   Ile Tyr Asn Leu Thr Leu Cys Glu Leu Asn Gly Thr Asp Trp Leu Ala
               20                  25                  30
   Asn Lys Phe Asp Trp Ala Val Glu Ser
           35                  40
<210> 12
   <211> 123
   <212> DNA
   <213> artificial
<220>
   <223> cDNA for the expression of aa 26-67 of the GP5 protein of the the North American PRRSV strain VR-2332
<400> 12
   gctgtgctcg ccaacgccag caacgacagc agctcccatc tacagctgat ttacaacttg 60
   acgctatgtg agctgaatgg cacagattgg ctagctaaca aatttgattg ggcagtggag 120
   agt 123
<210> 13
   <211> 141
   <212> PRT
   <213> artificial
<220>
   <223> aa 61-201 of the GP3 protein of the the North American PRRSV strain VR-2332
<400> 13
   Gln Ala Ala Ala Glu Ile Tyr Glu Pro Gly Arg Ser Leu Trp Cys Arg
   1               5                   10                  15
   Ile Gly Tyr Asp Arg Cys Glu Glu Asp Asp His Asp Glu Leu Gly Phe
               20                  25                  30
   Met Val Pro Pro Gly Leu Ser Ser Glu Gly His Leu Thr Ser Val Tyr
           35                  40                  45
   Ala Trp Leu Ala Phe Leu Ser Phe Ser Tyr Thr Ala Gln Phe His Pro
       50                  55                  60
   Glu Ile Phe Gly Ile Gly Asn Val Ser Arg Val Tyr Val Asp Ile Lys
   65                  70                  75                  80
   His Gln Leu Ile Cys Ala Glu His Asp Gly Gln Asn Thr Thr Leu Pro
   85 90 95
   Arg His Asp Asn Ile Ser Ala Val Phe Gln Thr Tyr Tyr Gln His Gln
   100 105 110
   Val Asp Gly Gly Asn Trp Phe His Leu Glu Trp Leu Arg Pro Phe Phe
   115 120 125
   Ser Ser Trp Leu Val Leu Asn Val Ser Trp Phe Leu Arg
   130 135 140
<210> 14
   <211> 423
   <212> DNA
   <213> artificial
<220>
   <223> cDNA for the expression of aa 61-201 of the GP3 protein of the the North American PRRSV strain VR-2332
<400> 14
   caagcagccg cagagatcta cgaacccggt aggtctcttt ggtgcaggat agggtatgac 60
   cgatgtgagg aggatgatca tgacgagcta gggtttatgg taccgcctgg cctctccagc 120
   gaaggccact tgactagtgt ttacgcctgg ttggcgttct tgtccttcag ctacacggcc 180
   cagttccatc ccgagatatt cgggataggg aatgtgagtc gagtttatgt tgacatcaaa 240
   catcaactca tctgcgccga acatgacggg cagaacacca ccttgcctcg tcatgacaac 300
   atttcagccg tgtttcagac ctattaccaa catcaagtcg acggcggcaa ttggtttcac 360
   ctagaatggc ttcgtccctt cttttcctcg tggttggttt taaatgtctc ttggtttctc 420
   agg 423
<210> 15
   <211> 78
   <212> PRT
   <213> artificial
<220>
   <223> aa 97-174 of the M protein of the the North American PRRSV strain VR-2332
<400> 15
   Ser Arg Cys Arg Leu Cys Leu Leu Gly Arg Lys Tyr Ile Leu Ala Pro
   1 5 10 15
   Ala His His Val Glu Ser Ala Ala Gly Phe His Pro Ile Ala Ala Asn
   20 25 30
   Asp Asn His Ala Phe Val Val Arg Arg Pro Gly Ser Thr Thr Val Asn
   35 40 45
   Gly Thr Leu Val Pro Gly Leu Lys Ser Leu Val Leu Gly Gly Arg Lys
   50 55 60
   Ala Val Lys Gln Gly Val Val Asn Leu Val Lys Tyr Ala Lys
   65 70 75
<210> 16
   <211> 234
   <212> DNA
   <213> artificial
<220>
   <223> cDNA for the expression of aa 97-174 of the M protein of the the North American PRRSV strain VR-2332
<400> 16
   tccagatgcc gtttgtgctt gctaggccgc aagtacattc tggcccctgc ccaccacgtt     60
   gaaagtgccg caggctttca tccgattgcg gcaaatgata accacgcatt tgtcgtccgg    120
   cgtcccggct ccactacggt caacggcaca ttggtgcccg ggttaaaaag cctcgtgttg    180
   ggtggcagaa aagctgttaa acagggagtg gtaaaccttg tcaaatatgc caaa          234
<210> 17
   <211> 128
   <212> PRT
   <213> artificial
<220>
   <223> ORF7 of the European strain of PRRSV (Lelystad Virus)
<400> 17
   Met Ala Gly Lys Asn Gln Ser Gln Lys Lys Lys Lys Ser Thr Ala Pro
   1               5                   10                  15
   Met Gly Asn Gly Gln Pro Val Asn Gln Leu Cys Gln Leu Leu Gly Ala
               20                  25                  30
   Met Ile Lys Ser Gln Arg Gln Gln Pro Arg Gly Gly Gln Ala Lys Lys
           35                  40                  45
   Lys Lys Pro Glu Lys Pro His Phe Pro Leu Ala Ala Glu Asp Asp Ile
       50                  55                  60
   Arg His His Leu Thr Gln Thr Glu Arg Ser Leu Cys Leu Gln Ser Ile
   65                  70                  75                  80
   Gln Thr Ala Phe Asn Gln Gly Ala Gly Thr Ala Ser Leu Ser Ser Ser
                   85                  90                  95
   Gly Lys Val Ser Phe Gln Val Glu Phe Met Leu Pro Val Ala His Thr
               100                 105                 110
   Val Arg Leu Ile Arg Val Thr Ser Thr Ser Ala Ser Gln Gly Ala Ser
           115                 120                 125
<210> 18
   <211> 388
   <212> DNA
   <213> artificial
<220>
   <223> cDNA for the expression of ORF7 of the European strain of PRRSV (Lelystad Virus)
<400> 18
   atggccggta aaaaccagag ccagaagaaa aagaaaagta cagctccgat ggggaatggc      60
   cagccagtca atcaactgtg ccagttgctg ggtgcaatga taaagtccca gcgccagcaa     120
   cctaggggag gacaggccaa aaagaaaaag cctgagaagc cacattttcc cctggctgct     180
   gaagatgaca tccggcacca cctcacccag actgaacgct ccctctgctt gcaatcgatc     240
   cagacggctt tcaatcaagg cgcaggaact gcgtcgcttt catccagcgg gaaggtcagt     300
   tttcaggttg agtttatgct gccggttgct catacagtgc gcctgattcg cgtgacttct     360
   acatccgcca gtcagggtgc aagttaat                                        388
<210> 19
   <211> 269
   <212> PRT
   <213> artificial
<220>
   <223> NSp7 of the European strain of PRRSV (Lelystad Virus)
<400> 19
   Ser Leu Thr Ala Ala Leu Ala Cys Lys Leu Ser Gln Ala Asp Leu Asp
   1               5                   10                  15
   Phe Leu Ser Ser Leu Thr Asn Phe Lys Cys Phe Val Ser Ala Ser Asn
               20                  25                  30
   Met Lys Asn Ala Ala Gly Gln Tyr Ile Glu Ala Ala Tyr Ala Lys Ala
           35                  40                  45
   Leu Arg Gln Glu Leu Ala Ser Leu Val Gln Ile Asp Lys Met Lys Gly
   50 55 60
   Val Leu Ser Lys Leu Glu Ala Phe Ala Glu Thr Ala Thr Pro Ser Leu
   65 70 75 80
   Asp Ile Gly Asp Val Ile Val Leu Leu Gly Gln His Pro His Gly Ser
   85 90 95
   Ile Leu Asp Ile Asn Val Gly Thr Glu Arg Lys Thr Val Ser Val Gln
   100 105 110
   Glu Thr Arg Ser Leu Gly Gly Ser Lys Phe Ser Val Cys Thr Val Val
   115 120 125
   Ser Asn Thr Pro Val Asp Ala Leu Thr Gly Ile Pro Leu Gln Thr Pro
   130 135 140
   Thr Pro Leu Phe Glu Asn Gly Pro Arg His Arg Ser Glu Glu Asp Asp
   145 150 155 160
   Leu Lys Val Glu Arg Met Lys Lys His Cys Val Ser Leu Gly Phe His
   165 170 175
   Asn Ile Asn Gly Lys Val Tyr Cys Lys Ile Trp Asp Lys Ser Thr Gly
   180 185 190
   Asp Thr Phe Tyr Thr Asp Asp Ser Arg Tyr Thr Gln Asp His Ala Phe
   195 200 205
   Gln Asp Arg Ser Ala Asp Tyr Arg Asp Arg Asp Tyr Glu Gly Val Gln
   210 215 220
   Thr Thr Pro Gln Gln Gly Phe Asp Pro Lys Ser Glu Thr Pro Val Gly
   225 230 235 240
   Thr Val Val Ile Gly Gly Ile Thr Tyr Asn Arg Tyr Leu Ile Lys Gly
   245 250 255
   Lys Glu Val Leu Val Pro Lys Pro Asp Asn Cys Leu Glu
   260 265
<210> 20
   <211> 807
   <212> DNA
   <213> artificial
<220>
   <223> cDNA for the expression of NSp7 of the European strain of PRRSV (Lelystad Virus)
<400> 20
   tccctaacgg ctgctttagc ttgcaagttg tcacaggctg accttgattt tttgtccagc      60
   ttaacgaact tcaagtgctt tgtatctgct tcaaacatga aaaatgctgc cggccagtac     120
   attgaagcag cgtatgccaa ggccctgcgc caagagttgg cctctctagt tcagattgac     180
   aaaatgaaag gagttttgtc caagctcgag gcctttgctg aaacagccac cccgtccctt     240
   gacataggtg acgtgattgt tctgcttggg caacatcctc acggatccat cctcgatatt     300
   aatgtgggga ctgaaaggaa aactgtgtcc gtgcaagaga cccggagcct aggcggctcc     360
   aaattcagtg tttgtactgt cgtgtccaac acacccgtgg acgccttgac cggcatccca     420
   ctccagacac caacccctct ttttgagaat ggtccgcgtc atcgcagcga ggaagacgat     480
   cttaaagtcg agaggatgaa gaaacactgt gtatccctcg gcttccacaa catcaatggc     540
   aaagtttact gcaaaatttg ggacaagtct accggtgaca ccttttacac ggatgattcc     600
   cggtacaccc aagaccatgc ttttcaggac aggtcagccg actacagaga cagggactat     660
   gagggtgtgc aaaccacccc ccaacaggga tttgatccaa agtctgaaac ccctgttggc     720
   actgttgtga tcggcggtat tacgtataac aggtatctga tcaaaggtaa ggaggttctg     780
   gtccccaagc ctgacaactg ccttgaa                                         807
<210> 21
   <211> 70
   <212> PRT
   <213> artificial
<220>
   <223> E protein of the European strain of PRRSV (Lelystad Virus)
<400> 21
   Met Gly Ser Leu Trp Ser Lys Ile Ser Gln Leu Phe Val Asp Ala Phe
   1               5                   10                  15
   Thr Glu Phe Leu Val Ser Val Val Asp Ile Val Ile Phe Leu Ala Ile
               20                  25                  30
   Leu Phe Gly Phe Thr Val Ala Gly Trp Leu Leu Val Phe Leu Leu Arg
           35                  40                  45
   Val Val Cys Ser Ala Leu Leu Arg Ser Arg Ser Ala Ile His Ser Pro
       50                  55                  60
   Glu Leu Ser Lys Val Leu
   65                  70
<210> 22
   <211> 210
   <212> DNA
   <213> artificial
<220>
   <223> cDNA for the expression of the E protein of the European strain of PRRSV (Lelystad Virus)
<400> 22
   atggggtcac tgtggagtaa aatcagccag ctgttcgtgg acgccttcac tgagttcctt     60
   gttagtgtgg ttgatattgt tattttcctt gccatactgt ttgggttcac cgtcgcagga    120
   tggttactgg tcttccttct cagagtggtt tgctccgcgc ttctccgttc gcgctctgcc    180
   attcactctc ccgagctatc gaaggtccta                                     210
<210> 23
   <211> 123
   <212> PRT
   <213> artificial
<220>
   <223> ORF7 of the North American PRRSV strain VR-2332
<400> 23
   Met Pro Asn Asn Asn Gly Lys Gln Gln Lys Arg Lys Lys Gly Asp Gly
   1               5                   10                  15
   Gln Pro Val Asn Gln Leu Cys Gln Met Leu Gly Lys Ile Ile Ala Gln
               20                  25                  30
   Gln Asn Gln Ser Arg Gly Lys Gly Pro Gly Lys Lys Asn Lys Lys Lys
           35                  40                  45
   Asn Pro Glu Lys Pro His Phe Pro Leu Ala Thr Glu Asp Asp Val Arg
       50                  55                  60
   His His Phe Thr Pro Ser Glu Arg Gln Leu Cys Leu Ser Ser Ile Gln
   65                  70                  75                  80
   Thr Ala Phe Asn Gln Gly Ala Gly Thr Cys Thr Leu Ser Asp Ser Gly
                   85                  90                  95
   Arg Ile Ser Tyr Thr Val Glu Phe Ser Leu Pro Thr His His Thr Val
               100                 105                 110
   Arg Leu Ile Arg Val Thr Ala Ser Pro Ser Ala
   115 120
<210> 24
   <211> 372
   <212> DNA
   <213> artificial
<220>
   <223> cDNA for the expression of ORF7 of the North American PRRSV strain VR-2332
<400> 24
   atgccaaata acaacggcaa gcagcagaat agaaagaagg gggatggcca gccagtcaat      60
   cagctgtgcc agatgctggg taagatcatc gctcagcaaa accagtccag aggcaaggga     120
   ccgggaaaga aaaataagaa gaaaaacccg gagaagcccc attttcctct agcgactgaa     180
   gatgatgtca gacatcactt tacccctagt gagcggcaat tgtgtctgtc gtcaatccag     240
   accgccttta atcaaggcgc tgggacttgc accctgtcag attcagggag gataagttac     300
   actgtggagt ttagtttgcc tacgcatcat actgtgcgcc tgattcgcgt cacagcatca     360
   ccctcagcat ga                                                          372
<210> 25
   <211> 259
   <212> PRT
   <213> artificial
<220>
   <223> NSp7 of the North American PRRSV strain VR-2332
<400> 25
   Ser Leu Thr Gly Ala Leu Ala Met Arg Leu Asn Asp Glu Asp Leu Asp
   1               5                   10                  15
   Phe Leu Met Lys Trp Thr Asp Phe Lys Cys Phe Val Ser Ala Ser Asn
               20                  25                  30
   Met Arg Asn Ala Ala Gly Gln Phe Ile Glu Ala Ala Tyr Ala Lys Ala
           35                  40                  45
   Leu Arg Val Glu Leu Ala Gln Leu Val Gln Val Asp Lys Val Arg Gly
       50                  55                  60
   Thr Leu Ala Lys Leu Glu Ala Phe Ala Asp Thr Val Ala Pro Gln Leu
   65                  70                  75                  80
   Ser Pro Gly Asp Ile Val Val Ala Leu Gly His Thr Pro Val Gly Ser
                   85                  90                  95
   Ile Phe Asp Leu Lys Val Gly Ser Thr Lys His Thr Leu Gln Ala Ile
   100 105 110
   Glu Thr Arg Val Leu Ala Gly Ser Lys Met Thr Val Ala Arg Val Val
   115 120 125
   Asp Pro Thr Pro Thr Pro Pro Pro Ala Pro Val Pro Ile Pro Leu Pro
   130 135 140
   Pro Lys Val Leu Glu Asn Gly Pro Asn Ala Trp Gly Asp Glu Asp Arg
   145 150 155 160
   Leu Asn Lys Lys Lys Arg Arg Arg Met Glu Ala Leu Gly Ile Tyr Val
   165 170 175
   Met Gly Gly Lys Lys Tyr Gln Lys Phe Trp Asp Lys Asn Ser Gly Asp
   180 185 190
   Val Phe Tyr Glu Glu Val His Asn Asn Thr Asp Glu Trp Glu Cys Leu
   195 200 205
   Arg Val Gly Asp Pro Ala Asp Phe Asp Pro Glu Lys Gly Thr Leu Cys
   210 215 220
   Gly His Val Thr Ile Glu Asn Lys Ala Tyr His Val Tyr Thr Ser Pro
   225 230 235 240
   Ser Gly Lys Lys Phe Leu Val Pro Val Asn Pro Glu Asn Gly Arg Val
   245 250 255
   Gln Trp Glu
<210> 26
   <211> 777
   <212> DNA
   <213> artificial
<220>
   <223> cDNA for the expression of NSp7 of the North American PRRSV strain VR-2332
<400> 26
   tctctgactg gtgccctcgc tatgagactc aatgacgagg acttggattt ccttatgaaa 60
   tggactgatt ttaagtgctt tgtttctgcg tccaacatga ggaatgcagc gggtcaattt 120
   atcgaggctg cctatgctaa agcacttaga gtagaactgg cccagttggt gcaggttgat 180
   aaagttcgag gtactttggc caaacttgaa gcttttgctg ataccgtggc acctcaactc 240
   tcgcccggtg acattgttgt cgctctcggc cacacgcctg ttggcagtat cttcgaccta 300
   aaggttggta gcaccaagca taccctccaa gccattgaga ccagagtcct tgctgggtcc 360
   aaaatgaccg tggcgcgcgt cgtcgacccg acccccacgc ccccacccgc acccgtgccc 420
   atccccctcc caccgaaagt tctggagaat ggccccaacg cttgggggga tgaggaccgt 480
   ttgaataaga agaagaggcg caggatggaa gccctcggca tctatgttat gggcgggaaa 540
   aagtaccaga aattttggga caagaattcc ggtgatgtgt tttatgagga ggtccataat 600
   aacacagatg agtgggagtg tctcagagtt ggcgaccctg ccgactttga ccctgagaag 660
   ggaactctgt gtggacatgt caccattgaa aacaaggctt accatgttta cacctcccca 720
   tctggtaaga agttcttggt ccccgtcaac ccagagaatg gaagagttca atgggaa 777
<210> 27
   <211> 6690
   <212> DNA
   <213> artificial
<220>
   <223> Vector pMal Tag2
<400> 27
   ccgacaccat cgaatggtgc aaaacctttc gcggtatggc atgatagcgc ccggaagaga 60
   gtcaattcag ggtggtgaat gtgaaaccag taacgttata cgatgtcgca gagtatgccg 120
   gtgtctctta tcagaccgtt tcccgcgtgg tgaaccaggc cagccacgtt tctgcgaaaa 180
   cgcgggaaaa agtggaagcg gcgatggcgg agctgaatta cattcccaac cgcgtggcac 240
   aacaactggc gggcaaacag tcgttgctga ttggcgttgc cacctccagt ctggccctgc 300
   acgcgccgtc gcaaattgtc gcggcgatta aatctcgcgc cgatcaactg ggtgccagcg 360
   tggtggtgtc gatggtagaa cgaagcggcg tcgaagcctg taaagcggcg gtgcacaatc 420
   ttctcgcgca acgcgtcagt gggctgatca ttaactatcc gctggatgac caggatgcca 480
   ttgctgtgga agctgcctgc actaatgttc cggcgttatt tcttgatgtc tctgaccaga 540
   cacccatcaa cagtattatt ttctcccatg aagacggtac gcgactgggc gtggagcatc 600
   tggtcgcatt gggtcaccag caaatcgcgc tgttagcggg cccattaagt tctgtctcgg 660
   cgcgtctgcg tctggctggc tggcataaat atctcactcg caatcaaatt cagccgatag 720
   cggaacggga aggcgactgg agtgccatgt ccggttttca acaaaccatg caaatgctga 780
   atgagggcat cgttcccact gcgatgctgg ttgccaacga tcagatggcg ctgggcgcaa 840
   tgcgcgccat taccgagtcc gggctgcgcg ttggtgcgga tatctcggta gtgggatacg 900
   acgataccga agacagctca tgttatatcc cgccgttaac caccatcaaa caggattttc 960
   gcctgctggg gcaaaccagc gtggaccgct tgctgcaact ctctcagggc caggcggtga 1020
   agggcaatca gctgttgccc gtctcactgg tgaaaagaaa aaccaccctg gcgcccaata 1080
   cgcaaaccgc ctctccccgc gcgttggccg attcattaat gcagctggca cgacaggttt 1140
   cccgactgga aagcgggcag tgagcgcaac gcaattaatg tgagttagct cactcattag 1200
   gcacaattct catgtttgac agcttatcat cgactgcacg gtgcaccaat gcttctggcg 1260
   tcaggcagcc atcggaagct gtggtatggc tgtgcaggtc gtaaatcact gcataattcg 1320
   tgtcgctcaa ggcgcactcc cgttctggat aatgtttttt gcgccgacat cataacggtt 1380
   ctggcaaata ttctgaaatg agctgttgac aattaatcat cggctcgtat aatgtgtgga 1440
   attgtgagcg gataacaatt tcacacagga aacagccagt ccgtttaggt gttttcacga 1500
   gcacttcacc aacaaggacc atagcatatg aaaatcgaag aaggtaaact ggtaatctgg 1560
   attaacggcg ataaaggcta taacggtctc gctgaagtcg gtaagaaatt cgagaaagat 1620
   accggaatta aagtcaccgt tgagcatccg gataaactgg aagagaaatt cccacaggtt 1680
   gcggcaactg gcgatggccc tgacattatc ttctgggcac acgaccgctt tggtggctac 1740
   gctcaatctg gcctgttggc tgaaatcacc ccggacaaag cgttccagga caagctgtat 1800
   ccgtttacct gggatgccgt acgttacaac ggcaagctga ttgcttaccc gatcgctgtt 1860
   gaagcgttat cgctgattta taacaaagat ctgctgccga acccgccaaa aacctgggaa 1920
   gagatcccgg cgctggataa agaactgaaa gcgaaaggta agagcgcgct gatgttcaac 1980
   ctgcaagaac cgtacttcac ctggccgctg attgctgctg acgggggtta tgcgttcaag 2040
   tatgaaaacg gcaagtacga cattaaagac gtgggcgtgg ataacgctgg cgcgaaagcg 2100
   ggtctgacct tcctggttga cctgattaaa aacaaacaca tgaatgcaga caccgattac 2160
   tccatcgcag aagctgcctt taataaaggc gaaacagcga tgaccatcaa cggcccgtgg 2220
   gcatggtcca acatcgacac cagcaaagtg aattatggtg taacggtact gccgaccttc 2280
   aagggtcaac catccaaacc gttcgttggc gtgctgagcg caggtattaa cgccgccagt 2340
   ccgaacaaag agctggcaaa agagttcctc gaaaactatc tgctgactga tgaaggtctg 2400
   gaagcggtta ataaagacaa accgctgggt gccgtagcgc tgaagtctta cgaggaagag 2460
   ttggcgaaag atccacgtat tgccgccact atggaaaacg cccagaaagg tgaaatcatg 2520
   ccgaacatcc cgcagatgtc cgctttctgg tatgccgtgc gtactgcggt gatcaacgcc 2580
   gccagcggtc gtcagactgt cgatgaagcc ctgaaagacg cgcagactaa ttcgagctcg 2640
   aacaacaaca acaataacaa taacaacaac ctcgggatcg agggaaggat ttcagaattc 2700
   ggatcctcta gagtcgacct gcagctcgag catcaccatc accatcacca tcaccattga 2760
   ctgcaggcaa gcttggcact ggccgtcgtt ttacaacgtc gtgactggga aaaccctggc 2820
   gttacccaac ttaatcgcct tgcagcacat ccccctttcg ccagctggcg taatagcgaa 2880
   gaggcccgca ccgatcgccc ttcccaacag ttgcgcagcc tgaatggcga atggcagctt 2940
   ggctgttttg gcggatgaga taagattttc agcctgatac agattaaatc agaacgcaga 3000
   agcggtctga taaaacagaa tttgcctggc ggcagtagcg cggtggtccc acctgacccc 3060
   atgccgaact cagaagtgaa acgccgtagc gccgatggta gtgtggggtc tccccatgcg 3120
   agagtaggga actgccaggc atcaaataaa acgaaaggct cagtcgaaag actgggcctt 3180
   tcgttttatc tgttgtttgt cggtgaacgc tctcctgagt aggacaaatc cgccgggagc 3240
   ggatttgaac gttgcgaagc aacggcccgg agggtggcgg gcaggacgcc cgccataaac 3300
   tgccaggcat caaattaagc agaaggccat cctgacggat ggcctttttg cgtttctaca 3360
   aactcttttt gtttattttt ctaaatacat tcaaatatgt atccgctcat gagacaataa 3420
   ccctgataaa tgcttcaata atattgaaaa aggaagagta tgagtattca acatttccgt 3480
   gtcgccctta ttcccttttt tgcggcattt tgccttcctg tttttgctca cccagaaacg 3540
   ctggtgaaag taaaagatgc tgaagatcag ttgggtgcac gagtgggtta catcgaactg 3600
   gatctcaaca gcggtaagat ccttgagagt tttcgccccg aagaacgttc tccaatgatg 3660
   agcactttta aagttctgct atgtggcgcg gtattatccc gtgttgacgc cgggcaagag 3720
   caactcggtc gccgcataca ctattctcag aatgacttgg ttgagtactc accagtcaca 3780
   gaaaagcatc ttacggatgg catgacagta agagaattat gcagtgctgc cataaccatg 3840
   agtgataaca ctgcggccaa cttacttctg acaacgatcg gaggaccgaa ggagctaacc 3900
   gcttttttgc acaacatggg ggatcatgta actcgccttg atcgttggga accggagctg 3960
   aatgaagcca taccaaacga cgagcgtgac accacgatgc ctgtagcaat ggcaacaacg 4020
   ttgcgcaaac tattaactgg cgaactactt actctagctt cccggcaaca attaatagac 4080
   tggatggagg cggataaagt tgcaggacca cttctgcgct cggcccttcc ggctggctgg 4140
   tttattgctg ataaatctgg agccggtgag cgtgggtctc gcggtatcat tgcagcactg 4200
   gggccagatg gtaagccctc ccgtatcgta gttatctaca cgacggggag tcaggcaact 4260
   atggatgaac gaaatagaca gatcgctgag ataggtgcct cactgattaa gcattggtaa 4320
   ctgtcagacc aagtttactc atatatactt tagattgatt taccccggtt gataatcaga 4380
   aaagccccaa aaacaggaag attgtataag caaatattta aattgtaaac gttaatattt 4440
   tgttaaaatt cgcgttaaat ttttgttaaa tcagctcatt ttttaaccaa taggccgaaa 4500
   tcggcaaaat cccttataaa tcaaaagaat agaccgagat agggttgagt gttgttccag 4560
   tttggaacaa gagtccacta ttaaagaacg tggactccaa cgtcaaaggg cgaaaaaccg 4620
   tctatcaggg cgatggccca ctacgtgaac catcacccaa atcaagtttt ttggggtcga 4680
   ggtgccgtaa agcactaaat cggaacccta aagggagccc ccgatttaga gcttgacggg 4740
   gaaagccggc gaacgtggcg agaaaggaag ggaagaaagc gaaaggagcg ggcgctaggg 4800
   cgctggcaag tgtagcggtc acgctgcgcg taaccaccac acccgccgcg cttaatgcgc 4860
   cgctacaggg cgcgtaaaag gatctaggtg aagatccttt ttgataatct catgaccaaa 4920
   atcccttaac gtgagttttc gttccactga gcgtcagacc ccgtagaaaa gatcaaagga 4980
   tcttcttgag atcctttttt tctgcgcgta atctgctgct tgcaaacaaa aaaaccaccg 5040
   ctaccagcgg tggtttgttt gccggatcaa gagctaccaa ctctttttcc gaaggtaact 5100
   ggcttcagca gagcgcagat accaaatact gtccttctag tgtagccgta gttaggccac 5160
   cacttcaaga actctgtagc accgcctaca tacctcgctc tgctaatcct gttaccagtg 5220
   gctgctgcca gtggcgataa gtcgtgtctt accgggttgg actcaagacg atagttaccg 5280
   gataaggcgc agcggtcggg ctgaacgggg ggttcgtgca cacagcccag cttggagcga 5340
   acgacctaca ccgaactgag atacctacag cgtgagctat gagaaagcgc cacgcttccc 5400
   gaagggagaa aggcggacag gtatccggta agcggcaggg tcggaacagg agagcgcacg 5460
   agggagcttc cagggggaaa cgcctggtat ctttatagtc ctgtcgggtt tcgccacctc 5520
   tgacttgagc gtcgattttt gtgatgctcg tcaggggggc ggagcctatg gaaaaacgcc 5580
   agcaacgcgg cctttttacg gttcctggcc ttttgctggc cttttgctca catgttcttt 5640
   cctgcgttat cccctgattc tgtggataac cgtattaccg cctttgagtg agctgatacc 5700
   gctcgccgca gccgaacgac cgagcgcagc gagtcagtga gcgaggaagc ggaagagcgc 5760
   ctgatgcggt attttctcct tacgcatctg tgcggtattt cacaccgcat atatggtgca 5820
   ctctcagtac aatctgctct gatgccgcat agttaagcca gtatacactc cgctatcgct 5880
   acgtgactgg gtcatggctg cgccccgaca cccgccaaca cccgctgacg cgccctgacg 5940
   ggcttgtctg ctcccggcat ccgcttacag acaagctgtg accgtctccg ggagctgcat 6000
   gtgtcagagg ttttcaccgt catcaccgaa acgcgcgagg cagctgcggt aaagctcatc 6060
   agcgtggtcg tgcagcgatt cacagatgtc tgcctgttca tccgcgtcca gctcgttgag 6120
   tttctccaga agcgttaatg tctggcttct gataaagcgg gccatgttaa gggcggtttt 6180
   ttcctgtttg gtcacttgat gcctccgtgt aagggggaat ttctgttcat gggggtaatg 6240
   ataccgatga aacgagagag gatgctcacg atacgggtta ctgatgatga acatgcccgg 6300
   ttactggaac gttgtgaggg taaacaactg gcggtatgga tgcggcggga ccagagaaaa 6360
   atcactcagg gtcaatgcca gcgcttcgtt aatacagatg taggtgttcc acagggtagc 6420
   cagcagcatc ctgcgatgca gatccggaac ataatggtgc agggcgctga cttccgcgtt 6480
   tccagacttt acgaaacacg gaaaccgaag accattcatg ttgttgctca ggtcgcagac 6540
   gttttgcagc agcagtcgct tcacgttcgc tcgcgtatcg gtgattcatt ctgctaacca 6600
   gtaaggcaac cccgccagcc tagccgggtc ctcaacgaca ggagcacgat catgcgcacc 6660
   cgtggccagg acccaacgct gcccgaaatt 6690
<210> 28
   <211> 462
   <212> PRT
   <213> artificial
<220>
   <223> aa sequence of maltose binding protein-lacZ alpha peptide fusion protein precursor [Shuttle vector pMAL-pIII] (E.coli; GenBank accession AAB86559)
<400> 28
   Met Lys Ile Glu Glu Gly Lys Leu Val Ile Trp Ile Asn Gly Asp Lys
   1 5 10 15
   Gly Tyr Asn Gly Leu Ala Glu Val Gly Lys Lys Phe Glu Lys Asp Thr
   20 25 30
   Gly Ile Lys Val Thr Val Glu His Pro Asp Lys Leu Glu Glu Lys Phe
   35 40 45
   Pro Gln Val Ala Ala Thr Gly Asp Gly Pro Asp Ile Ile Phe Trp Ala
   50 55 60
   His Asp Arg Phe Gly Gly Tyr Ala Gln Ser Gly Leu Leu Ala Glu Ile
   65 70 75 80
   Thr Pro Asp Lys Ala Phe Gln Asp Lys Leu Tyr Pro Phe Thr Trp Asp
   85 90 95
   Ala Val Arg Tyr Asn Gly Lys Leu Ile Ala Tyr Pro Ile Ala Val Glu
   100 105 110
   Ala Leu Ser Leu Ile Tyr Asn Lys Asp Leu Leu Pro Asn Pro Pro Lys
   115 120 125
   Thr Trp Glu Glu Ile Pro Ala Leu Asp Lys Glu Leu Lys Ala Lys Gly
   130 135 140
   Lys Ser Ala Leu Met Phe Asn Leu Gln Glu Pro Tyr Phe Thr Trp Pro
   145 150 155 160
   Leu Ile Ala Ala Asp Gly Gly Tyr Ala Phe Lys Tyr Glu Asn Gly Lys
   165 170 175
   Tyr Asp Ile Lys Asp Val Gly Val Asp Asn Ala Gly Ala Lys Ala Gly
   180 185 190
   Leu Thr Phe Leu Val Asp Leu Ile Lys Asn Lys His Met Asn Ala Asp
   195 200 205
   Thr Asp Tyr Ser Ile Ala Glu Ala Ala Phe Asn Lys Gly Glu Thr Ala
   210 215 220
   Met Thr Ile Asn Gly Pro Trp Ala Trp Ser Asn Ile Asp Thr Ser Lys
   225 230 235 240
   Val Asn Tyr Gly Val Thr Val Leu Pro Thr Phe Lys Gly Gln Pro Ser
   245 250 255
   Lys Pro Phe Val Gly Val Leu Ser Ala Gly Ile Asn Ala Ala Ser Pro
   260 265 270
   Asn Lys Glu Leu Ala Lys Glu Phe Leu Glu Asn Tyr Leu Leu Thr Asp
   275 280 285
   Glu Gly Leu Glu Ala Val Asn Lys Asp Lys Pro Leu Gly Ala Val Ala
   290 295 300
   Leu Lys Ser Tyr Glu Glu Glu Leu Ala Lys Asp Pro Arg Ile Ala Ala
   305 310 315 320
   Thr Met Glu Asn Ala Gln Lys Gly Glu Ile Met Pro Asn Ile Pro Gln
   325 330 335
   Met Ser Ala Phe Trp Tyr Ala Val Arg Thr Ala Val Ile Asn Ala Ala
   340 345 350
   Ser Gly Arg Gln Thr Val Asp Glu Ala Leu Lys Asp Ala Gln Thr Asn
   355 360 365
   Ser Ser Ser Asn Asn Asn Asn Asn Asn Asn Asn Asn Asn Leu Gly Ile
   370 375 380
   Glu Gly Arg Ile Ser Glu Phe Gly Ser Ser Arg Val Asp Leu Gln Ala
   385 390 395 400
   Ser Leu Ala Leu Ala Val Val Leu Gln Arg Arg Asp Trp Glu Asn Pro
                   405                 410                 415
   Gly Val Thr Gln Leu Asn Arg Leu Ala Ala His Pro Pro Phe Ala Ser
               420                 425                 430
   Trp Arg Asn Ser Glu Glu Ala Arg Thr Asp Arg Pro Ser Gln Gln Leu
           435                 440                 445
   Arg Ser Leu Asn Gly Glu Trp Gln Leu Gly Cys Phe Gly Gly
       450                 455                 460